(19) Europäisches Patentamt
European Patent Office
Office européen des brevets

(11) Publication number : 0 268 688 B1

# (12) EUROPEAN PATENT SPECIFICATION

(45) Date of publication of patent specification : **04.03.92 Bulletin 92/10**

(21) Application number : **87903425.4**

(22) Date of filing : **26.05.87**

(86) International application number : **PCT/JP87/00334**

(87) International publication number : **WO 87/07268 03.12.87 Gazette 87/27**

(51) Int. Cl.[5] : **C07C 275/64,** C07C 275/06, C07D 213/63, C07D 239/34, C07D 241/18, C07D 251/30, C07D 295/125, A01N 47/28, A01N 47/32

## (54) SUBSTITUTED ARYLOXYUREAS, PROCESS FOR THEIR PREPARATION, AND THEIR USE.

(30) Priority : **26.05.86 JP 119293/86**

(43) Date of publication of application : **01.06.88 Bulletin 88/22**

(45) Publication of the grant of the patent : **04.03.92 Bulletin 92/10**

(84) Designated Contracting States : **AT BE CH DE FR GB IT LI NL**

(56) References cited :
**EP-A- 0 183 174**
**JP-A-61 126 065**
**JP-B- 415 254**
**US-A- 3 332 975**

(73) Proprietor : **MITSUI PETROCHEMICAL INDUSTRIES, LTD.**
**2-5, Kasumigaseki 3-chome Chiyoda-ku**
**Tokyo 100 (JP)**
Proprietor : **KUMIAI CHEMICAL INDUSTRY CO., LTD.**
**4-26, Ikenohata 1-chome**
**Taitoh-ku Tokyo 110 (JP)**

(72) Inventor : **HASHIMOTO, Isao**
**37-25, Hirata 6-chome**
**Iwakuni-shi Yamaguchi 741 (JP)**
Inventor : **ISHIDA, Tatsuyoshi**
**2-7, Misono 1-chome**
**Ohtake-shi Hiroshima 739-06 (JP)**
Inventor : **TSURU, Kazutaka**
**12-2, Shouzoku-cho 5-chome**
**Iwakuni-shi Yamaguchi 740 (JP)**
Inventor : **YAMADA, Yuji**
**3353, Kamo**
**Kikugawa-cho, Ogasa-gun Shizuoka 439 (JP)**
Inventor : **MIYAZAWA, Takeshige**
**1809, Kamo**
**Kikugawa-cho, Ogasa-gun Shizuoka 439 (JP)**
Inventor : **NAKAMURA, Yasuo**
**14-15, Aobadai 1-chome**
**Kikugawa-cho, Ogasa-gun Shizuoka 439 (JP)**

(74) Representative : **Wächtershäuser, Günter, Dr.**
**Tal 29**
**W-8000 München 2 (DE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 268 688 B1

## Description

This invention relates to a novel substituted aryloxyurea, processes for its production, and to a herbicide comprising it as an active ingredient.

## BACKGROUND TECHNOLOGY

Many herbicides have been used to secure quantities of crops harvested. Urea-series compounds, for example DCMU widely used as a herbicide for upland farms, have the defect that they cannot be used in paddies because of their strong phytotoxicity.

U. S. Patent Specification No. 3,332,975 discloses that m-chlorophenoxyureas which differ in chemical structure from the compounds of this invention can be used as medicines. As will be stated hereinafter, however, these compounds do not exhibit a herbicidal efficacy, and even if they do, their herbicidal efficacy is very weak.

Japanese Patent Publication No. 5254/1966 describes substituted ureas or thioureas represented by the following formula

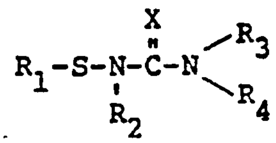

wherein $R_1$ represents a phenyl or halophenyl group, or a group of the formula

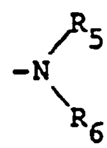

in which $R_5$ and $R_6$ are the same alkyl or aralkyl groups, or may form a piperidino or morpholino group together with the nitrogen atom to which they are bonded; $R_2$ represents a lower aliphatic hydrocarbon group, a phenyl group, or a phenyl group substituted by at least one of halogen, alkylmercapto, nitro and alkoxy; $R_3$ and $R_4$ are the same alkyl or aralkyl groups, or may form a piperidino or morpholino group together with the nitrogen atom to which they are bonded; and X is oxygen or sulfur.
It also describes that these compounds are herbicides, although it discloses no biological data.

Japanese Patent Publication No. 6999/1971 discloses that compounds of the following formula

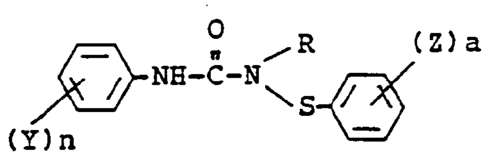

wherein R is a $C_1$-$C_4$ alkyl group; Y is F, Cl, Br or 1, a $C_1$-$C_4$ alkyl group, or a $C_1$-$C_4$ alkoxy group; n is 0, or an integer of 1 to 3 when Y is the halogen atom or 1 when Y is the alkyl or alkoxy group; Z is F, Cl, Br or I or a $C_1$-$C_4$ alkyl group; and a is 0, or an integer of 1 to 5 when Z is the halogen atom, or 1 when Z is the alkyl group, are a herbicide to be applied to plants or their growing sites.

European Patent Application EP-183174, a prior application with respect to the present application, disclose compounds represented by the following formula (I)

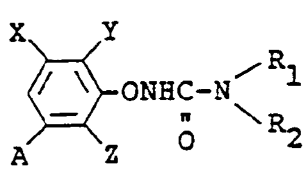

(I)

wherein A is a halogen atom or a trifluoromethyl group; X, Y and Z each represent a hydrogen atom, a halogen atom or a trifluoromethyl group; $R_1$ represents a $C_1$-$C_6$ alkyl group, a lower alkoxy group, a lower alkoxy-substituted lower alkyl group, a cycloalkyl-substituted lower alkyl group, a lower alkenyl group, a lower alkynyl group, a lower haloalkyl group, a lower haloalkenyl group, or a $C_3$-$C_9$ non-aromatic cyclic hydrocarbon group; $R_2$ is a hydrogen atom, a $C_1$-$C_6$ alkyl group, a lower alkenyl group or a lower alkynyl group; and $R_1$ and $R_2$, together with the nitrogen atom to which they are bonded, may form a 3- to 8-membered (which may be a bicyclic ring) which may contain a double bond or an oxygen atom in the ring or at least one branch.
They state that these compounds have a herbicidal efficacy.

It is an object of this invention to provide novel substituted aryloxyureas.

Another object of this invention is to provide novel substituted aryloxyureas which exhibit an excellent herbicidal efficacy.

Still another object of this invention is to provide novel substituted aryloxyureas which exhibit an excellent herbicidal efficacy against weeds when applied to the weeds over a wide range of period from a pre-emergence stage to a growing stage.

Yet another object of this invention is to provide novel substituted aryloxyureas which exhibit a herbicidal efficacy and yet are highly safe to crops or crop plants.

A further object of this invention is to produce the compounds of this invention having excellent herbicidal activity with industrial advantage.

Other objects and advantages of this invention will become apparent from the following description.

## DISCLOSURE OF THE INVENTION

According to this invention, the above objects and advantages of this invention are achieved by substituted aryloxyureas represented by the following formula (I)

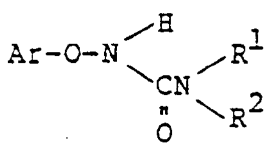

.... (I)

wherein Ar represents a group selected from the class consisting of aryl groups of the following formulae

(a)

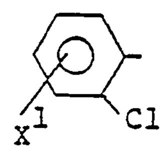

in which $X^1$ represents a hydrogen atom, a $C_1$-$C_4$ alkyl group or a $C_1$-$C_4$ alkoxy group,

(b)

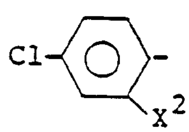

in which $X^2$ represents a hydrogen or halogen atom,

(c)

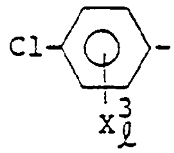

in which $X^3$ represents a halogen atom and $\ell$ is an integer of 2 or 3,

(d)

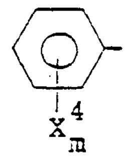

in which $X^4$ represents a fluorine, bromine or iodine atom, and m is an integer of 1, 2 or 3,

(e)

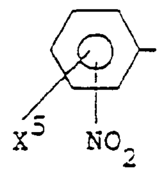

in which $X^5$ represents a hydrogen atom, a halogen atom a $C_1$-$C_4$ alkyl group, a $C_1$-$C_4$ alkoxy group or a trifluoromethyl group

(f)

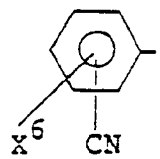

in which $X^6$ represents a hydrogen or halogen atom,

(g)

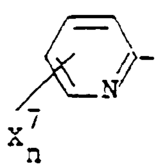

in which $X^7$ represents a hydrogen atom, a halogen atom, a cyano group, a $C_1$-$C_4$ alkoxy group or a trifluoromethyl group, and n is an integer of 1 or 2,

(h)

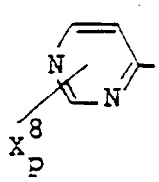

in which $X^8$ represents a hydrogen atom, a chlorine atom or a $C_1$-$C_4$ alkylthio group, and p is an integer of 1 or 2,

(i)

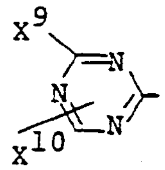

in which $X^9$ and $X^{10}$ are identical or different and each represents a chlorine atom, a $C_1$-$C_4$ alkoxy group or a $C_1$-$C_4$ alkylthio group, and

(j)

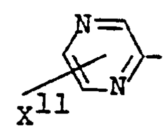

in which $X^{11}$ represents a hydrogen or chlorine atom;

$R^1$ represents a $C_1$-$C_6$ alkyl group, a $C_3$-$C_5$ alkenyl group, a $C_3$-$C_5$ alkynyl group or a $C_3$-$C_7$ cycloalkyl group; $R^2$ represents a hydrogen atom or a $C_1$-$C_6$ alkyl group; and $R^1$ and $R^2$, together with the nitrogen atom to which they are bonded, may form a 4- to 8-membered heterocyclic ring which may contain an oxygen atom as a ring member or may be substituted by a $C_1$-$C_4$ alkyl group or a $C_2$-$C_4$ alkylene group, whith the proviso, that Ar does not represent 2,5-difluorophenyl, when $R^1$ is cyclopentyl or cyclobenzyl and $R^2$ is a hydrogen atom, and acid addition salts thereof.

In formula (I), Ar is a group of any of the formulae (a) to (j) above. The groups of formulae (a) to (j) are substituted phenyl [(a) to (f)], pyridyl (g), pyrimidinyl (h), triazinyl (i) and pyrazinyl (j) groups, and are common in that they are aromatic groups.

In the group of formula (a), $X^1$ is a hydrogen atom, a $C_1$-$C_4$ alkyl group or a $C_1$-$C_4$ alkoxy group.

The $C_1$-$C_4$ alkyl group is a linear or branched alkyl group having 1 to 4 carbon atoms, such as methyl, ethyl, n-propyl, iso-propyl, n-butyl, sec-butyl, iso-butyl or t-butyl.

The $C_1$-$C_4$ alkoxy group is one having a linear or branched alkyl moiety with 1 to 4 carbon atoms. Examples are methoxy, ethoxy, n-propoxy, isopropoxy, n-butoxy, sec-butoxy, iso-butoxy, and t-butoxy.

Preferred examples of the group of formula (a) are 2-chlorophenyl, 2-chloro-5-methylphenyl, 2-chloro-3-methylphenyl, 2-chloro-4-methylphenyl, 2-chloro-6-methylphenyl, 2-chloro-5-methoxyphenyl, 2-chloro-6-methoxyphenyl, 2-chloro-4-methoxyphenyl and 2-chloro-3-methoxyphenyl.

In formula (b), $X^2$ represents a hydrogen or halogen atom.

Preferred examples of the halogen atom are fluorine, chlorine, bromine and iodine.

Preferred examples of the group (b) are 4-chlorophenyl, 2,4-dichlorophenyl, 2-bromo-4-chlorophenyl and 4-chloro-2-fluorophenyl.

In formula (c), $X^3$ is a halogen atom, and $\ell$ is an integer of 2 or 3.

Fluorine, chlorine, bromine and iodine may be cited as preferred examples of the halogen atom.

Preferred examples of the group of formula (c) are 2,4,5-trichlorophenyl, 3,4,5-trichlorophenyl, 2,3,4-trichlorophenyl, 2,4,6-trichlorophenyl and 2,3,4,5-tetrachlorophenyl.

In formula (d), $X^4$ represents a fluorine, bromine or iodine atom, and m is an integer of 1, 2 or 3.

Preferred examples of the group of formula (d) include 2-fluorophenyl, 3-fluorophenyl, 4-fluorophenyl, 2,5-difluorophenyl, 3,5-difluorophenyl, 2,4-difluorophenyl, 4-bromophenyl, 3-bromophenyl, 2-bromophenyl, 2-iodophenyl and 4-iodophenyl.

In formula (e), $X^5$ represents a hydrogen atom, a halogen atom, a $C_1$-$C_4$ alkyl group, a $C_1$-$C_4$ alkoxy group, or a trifluoromethyl group.

Examples of the halogen atom may be the same as those given above with regard to $X^3$.

Specific examples of the $C_1$-$C_4$ alkyl and $C_1$-$C_4$ alkoxy groups are the same as those given above with regard to $X^1$.

Examples of formula (e) include 2-nitrophenyl, 3-nitrophenyl, 4-nitrophenyl, 4-chloro-2-nitrophenyl, 5-chloro-2-nitrophenyl, 5-chloro-2-nitrophenyl, 3-chloro-5-nitrophenyl, 5-methyl-2-nitrophenyl, 4-methyl-2-nitrophenyl, 3-chloro-4-nitrophenyl, 2-chloro-4-nitrophenyl, 2-methyl-6-nitrophenyl, 3-methyl-4-nitrophenyl, 2-methyl-4-nitrophenyl, 2-methyl-5-nitrophenyl, 2-methoxy-5-nitrophenyl, 2-nitro-4-trifluoromethylphenyl, 3-nitro-5-trifluoromethylphenyl and 5-nitro-2-trifluoromethyl.

In formula (f), $X^6$ is a hydrogen or halogen atom.

Preferred specific examples of the halogen atom may be the same as those given with regard to $X^3$.

Preferred examples of the group of formula (f) are 2-cyanophenyl, 3-cyanophenyl, 4-cyanophenyl, 3-chloro-2-cyanophenyl, 4-chloro-2-cyanophenyl, 5-chloro-2-cyanophenyl, 6-chloro-2-cyanophenyl, 2-chloro-3-cyanophenyl, 4-chloro-3-cyanophenyl, 3-chloro-5-cyanophenyl, 2-chloro-5-cyanophenyl, 3-chloro-4-cyanophenyl and 2-chloro-4-cyanophenyl.

In formula (g), $X^7$ is a hydrogen atom, a halogen atom, a cyano group, a $C_1$-$C_4$ alkoxy group or a trifluoromethyl group.

Preferred specific examples of the halogen atom and the $C_1$-$C_4$ alkoxy groups may be the same as those given with regard to $X^5$.

Examples of the group of formula (g) are 6-chloro-2-pyridyl, 5-chloro-2-pyridyl, 4-chloro-2-pyridyl, 3-chloro-2-pyridyl, 3,5-dichloro-2-pyridyl, 6-methoxy-2-pyridyl, 5-methoxy-2-pyridyl, 4-methoxy-2-pyridyl, 3-methoxy-2-pyridyl, 4-trifluoromethyl-2-pyridyl, 5-trifluoromethyl-2-pyridyl, 6-trifluoromethyl-2-pyridyl and 3-chloro-5-trifluoromethyl-2-pyridyl. Of these, 6-chloro-2-pyridyl and 6-trifluoromethyl-2-pyridyl are preferred.

In formula (h), $X^8$ is a hydrogen atom, a chlorine atom, or a $C_1$-$C_4$ alkylthio group, and p is an integer of 1 or 2.

The $C_1$-$C_4$ alkylthio is an alkylthio group having a linear or branched alkyl moiety with 1 to 4 carbon atoms. Preferred examples are methylthio, ethylthio, n-propylthio, iso-propylthio, n-butylthio, iso-butylthio, sec-butylthio and t-butylthio.

Preferred examples of formula (h) are 4-pyrimidinyl, 2-chloro-4-pyrimidinyl, 6-chloro-2-pyrimidinyl, 2,6-dichloro-4-pyrimidinyl, 6-chloro-2-methylthio-4-pyrimidinyl and 2-methylthio-4-pyrimidinyl.

In formula (i), $X^9$ and $X^{10}$ are identical or different and each represents a chlorine atom, a $C_1$-$C_4$ alkoxy group or a $C_1$-$C_4$ alkylthio group.

Preferred specific examples of the $C_1$-$C_4$ alkoxy group may be the same as those given with regard to $X^1$.

Preferred specific examples of the $C_1$-$C_4$ alkylthio group may be the same as those given with regard to $X^8$.

Examples of the group of formula (i) are 4-chloro-6-methoxy-2-triazinyl, 4,6-dimethoxy-2-triazinyl, 4-methoxy-6-methylthio-2-triazinyl, 4-chloro-6-methylthio-2-triazinyl and 4,6-dimethylthio-2-triazinyl.

In formula (j), $X^{11}$ is a hydrogen or chlorine atom. Preferred examples of formula (j) are 2-pyrazinyl, 3-chloro-2-pyrazinyl, 5-chloro-2-pyrazinyl and 6-chloro-2-pyrazinyl groups.

The groups of formulae (a), (d), (e), (g), (h) and (j) are preferred as Ar.

In formula (I), $R^1$ is a $C_1$-$C_6$ alkyl group, a $C_3$-$C_5$ alkenyl group, a $C_3$-$C_5$ alkynyl group or a $C_3$-$C_7$ cycloalkyl group. The $C_1$-$C_6$ alkyl and $C_3$-$C_7$ cycloalkyl groups are preferred.

The $C_1$-$C_6$ alkyl group may be linear or branched, and examples may include methyl, ethyl, n-propyl, isopropyl, n-butyl, isobutyl, sec-butyl, tert-butyl, n-amyl, isoamyl, sec-amyl, active amyl, tert-amyl, 1,2-dimethylpropyl, 2,2-dimethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1,2,2-trimethylpropyl, 1-ethylpropyl and 2-ethylbutyl groups.

Unsaturated hydrocarbon groups having 3 to 5 carbon atoms in the longest carbon chain portion are preferred as the $C_3$-$C_5$ alkenyl group. Examples include allyl, 2-butenyl, 3-butenyl, 1-methyl-2-propenyl, 2-methyl-2-propenyl, 1,2-dimethyl-2-propenyl, 2-methyl-2-butenyl, 3-methyl-2-butenyl and 2,3-dimethyl-2-butenyl groups.

Unsaturated hydrocarbon groups having 3 to 5 carbon atoms in the longest carbon chain portion are preferred as the $C_3$-$C_5$ alkenyl group. Examples include 2-propynyl, 2-butynyl, 3-butynyl, 1-methyl-2-propynyl and 1,1-dimethyl-2-propynyl groups.

Examples of the $C_3$-$C_7$ cycloalkyl group are cyclopropyl, cyclobutyl, cyclopentyl, cyclohexyl and cycloheptyl.

The $C_1$-$C_4$ alkyl and $C_3$-$C_7$ cycloalkyl groups are especially preferred as $R^1$.

In formula (I), $R^2$ is a hydrogen atom or a $C_1$-$C_6$ alkyl group. The $C_1$-$C_6$ group is preferred.

Examples of the $C_1$-$C_4$ alkyl group may be the same as those given above with regard to $R^1$.

$R^1$ and $R^2$, taken together, may form a 4- to 8-membered, preferably 6- to 7-membered heterocyclic group together with the nitrogen atom to which they are bonded. The heterocyclic group may contain an oxygen atom as a ring member or may be substituted by a $C_1$-$C_4$ alkyl group or a $C_2$-$C_4$ alkylene group. Examples of the $C_1$-$C_4$ alkyl substituent may be the same as those given above with regard to $R^1$. Alkylene groups having 2 to 4 carbon atoms as the $C_2$-$C_4$ alkylene substituent are example ethylene, trimethylene and tetramethylene groups.

Examples of the heterocyclic group are given below.

Four-membered heterocyclic groups:

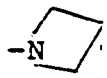 , 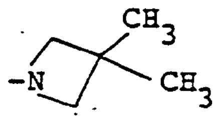 .

Five-membered heterocyclic groups:

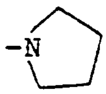 , 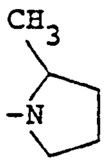 , 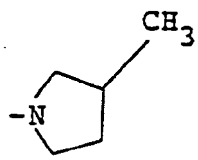 , 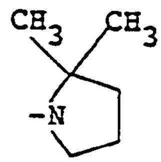 , 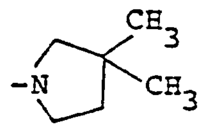 ,

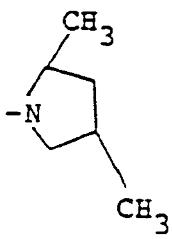 , 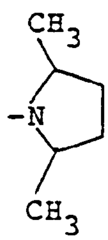 , 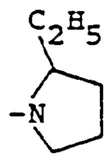 , 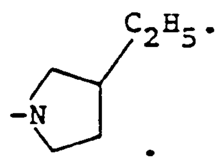 .

Six-membered heterocyclic groups:

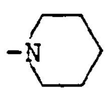 , 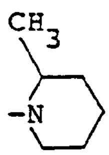 , 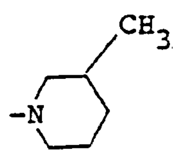 , 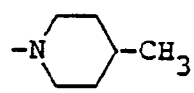 , 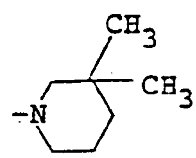 ,

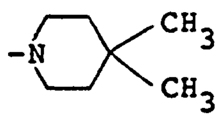 , 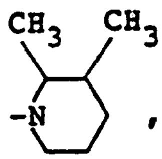 , 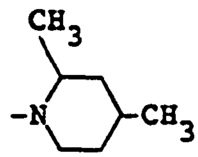 , 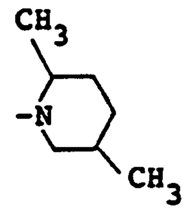

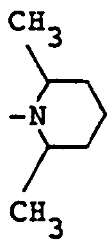 , 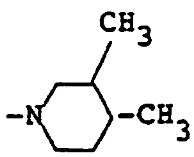 , 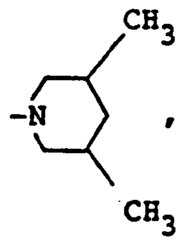 , 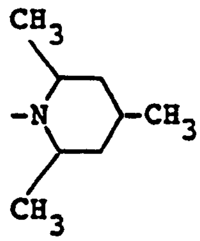 , 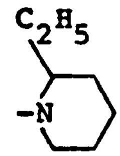 ,

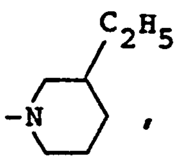 , 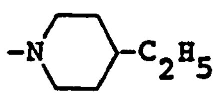 , 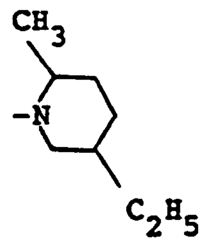 , 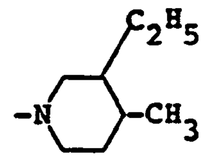

Seven-membered cyclic groups:

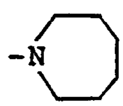 , 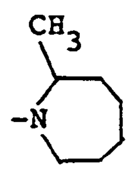 , 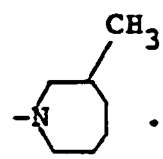 .

Eight-membered hefterocyclic groups:

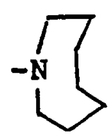

Heterocyclic groups containing an oxygen atom:

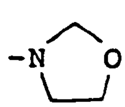 , 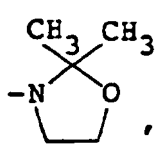 , 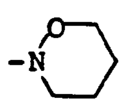 , 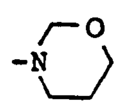 , 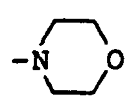 ,

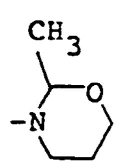 , 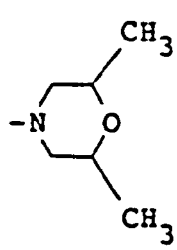 .

Heterocyclic groups (bicyclic groups) containing lower alkylene groups:

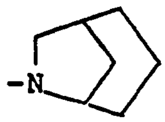 , 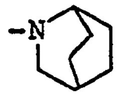 , 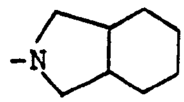 , 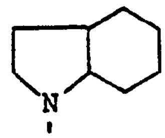 ,

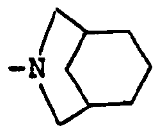 , 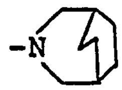 , 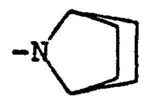 , 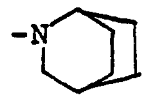 .

The compounds given in Table 1 below are preferred examples of the compounds of formula (I) provided by the invention.

Table 1

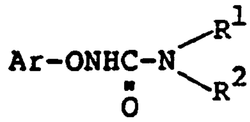

| Compound No. | Ar | $R^1$ | $R^2$ |
|---|---|---|---|
| 1 | 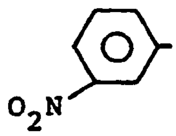 | 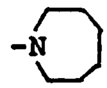 | |
| 2 | 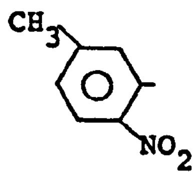 | 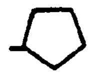 | H |
| 3 | 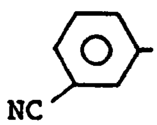 | 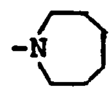 | |
| 4 | 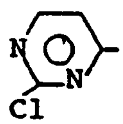 | 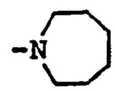 | |
| 5 | 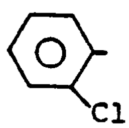 | $CH_3$ | $CH_3$ |
| 6 | " | 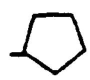 | H |
| 7 | " | 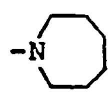 | |

- to be continued -

Table 1 (continued)

| Compound No. | Ar | $R^1$ | $R^2$ |
|---|---|---|---|
| 8 | 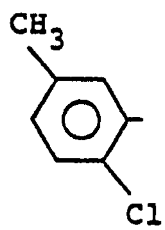 | 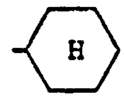 | H |
| 9 | " | 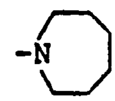 | |
| 10 | 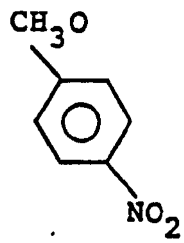 | 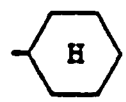 | H |
| 11 | " | 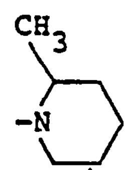 | |
| 12 | 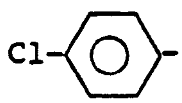 | $CH_3$ | $CH_3$ |
| 13 | 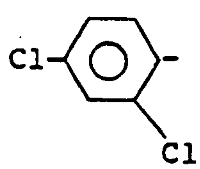 | $CH_3$ | $CH_3$ |
| 14 | " | 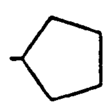 | H |

- to be continued -

Table 1 (continued)

| Compound No. | Ar | $R^1$ | $R^2$ |
|---|---|---|---|
| 15 | 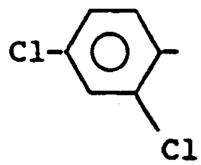 | 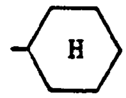 | H |
| 16 | " | 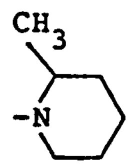 | |
| 17 | " | 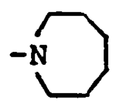 | |
| 18 | 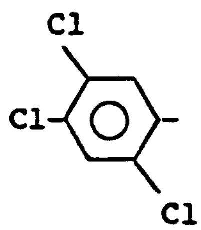 | $CH_3$ | $CH_3$ |
| 19 | 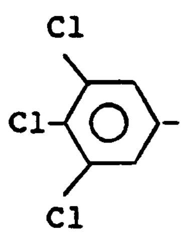 | $CH_3$ | $CH_3$ |
| 20 | 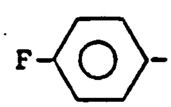 | $CH_3$ | $CH_3$ |
| 21 | 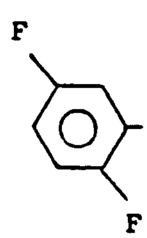 | 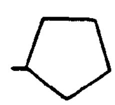 | H |

- to be continued -

Table 1 (continued)

| Compound No. | Ar | $R^1$ | $R^2$ |
|---|---|---|---|
| 22 | 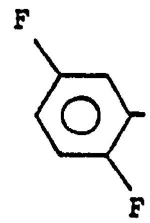 | 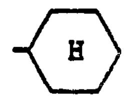 | H |
| 23 | 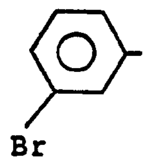 | 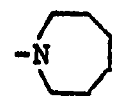 | |
| 24 | 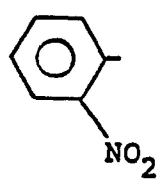 | $CH_3$ | $CH_3$ |
| 25 | " | 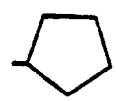 | H |
| 26 | " | $-CH_2C\equiv CH$ | $CH_3$ |
| 27 | 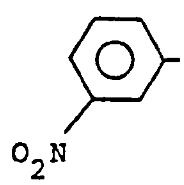 | $CH_3$ | $CH_3$ |
| 28 | " | $-CH_2CH=CH_2$ | $CH_3$ |
| 29 | " | 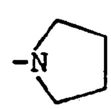 | |

- to be continued -

Table 1 (continued)

| Compound No. | Ar | $R^1$ | $R^2$ |
|---|---|---|---|
| 30 | 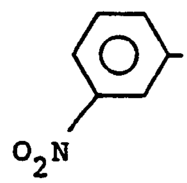 | 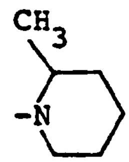 | |
| 31 | 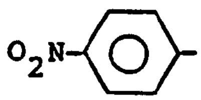 | $CH_3$ | $CH_3$ |
| 32 | 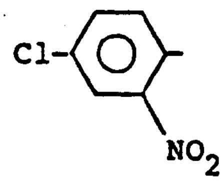 | 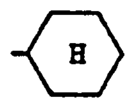 | H |
| 33 | 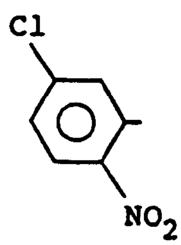 | $n-C_3H_7$ | H |
| 34 | " | $i-C_3H_7$ | H |
| 35 | " | $-CH_2CH=CH_2$ | H |
| 36 | " | $sec-C_4H_9$ | H |
| 37 | " | $t-C_4H_9$ | H |

- to be continued -

Table 1 (continued)

| Compound No. | Ar | $R^1$ | $R^2$ |
|---|---|---|---|
| 38 | 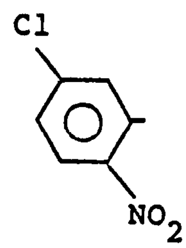 | 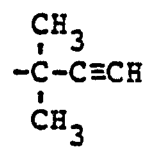 | H |
| 39 | " | 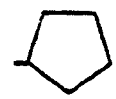 | H |
| 40 | " | 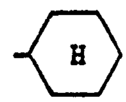 | H |
| 41 | 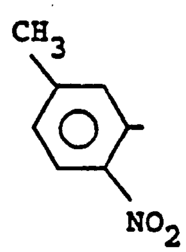 | $CH_3$ | $CH_3$ |
| 42 | " | 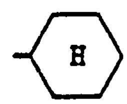 | H |
| 43 | " | 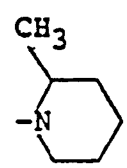 | |
| 44 | " | 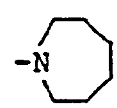 | |
| 45 | 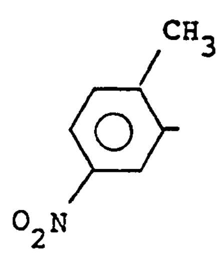 | $CH_3$ | $CH_3$ |

- to be continued -

Table 1 (continued)

| Compound No. | Ar | $R^1$ | $R^2$ |
|---|---|---|---|
| 46 | 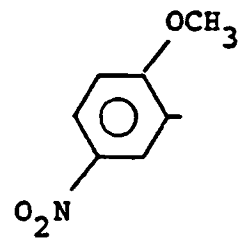 | 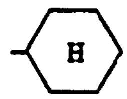 | H |
| 47 | " | 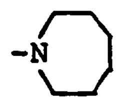 | |
| 48 | 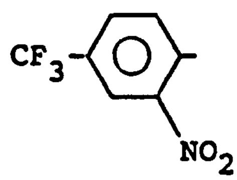 | 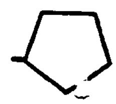 | H |
| 49 | 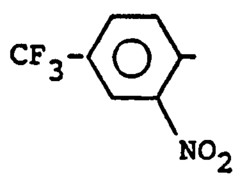 | 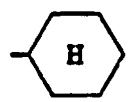 | H |
| 50 | 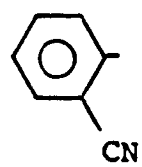 | 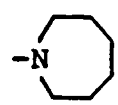 | |
| 51 | 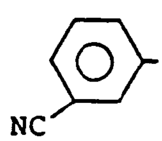 | 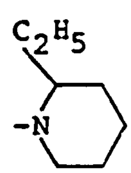 | |
| 52 | 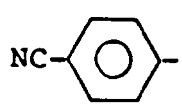 | $CH_3$ | $CH_3$ |

- to be continued -

Table 1 (continued)

| Compound No. | Ar | $R^1$ | $R^2$ |
|---|---|---|---|
| 53 | 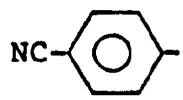 | 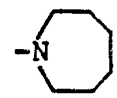 | |
| 54 | 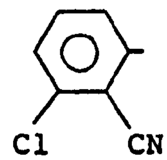 | 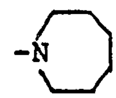 | |
| 55 | 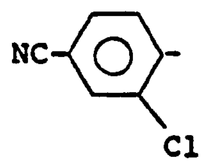 | sec-$C_4H_9$ | H |
| 56 | 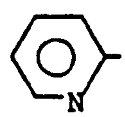 | 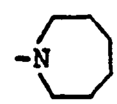 | |
| 57 | 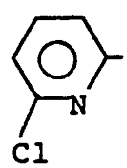 | $CH_3$ | $CH_3$ |
| 58 | " | sec-$C_4H_9$ | H |
| 59 | " | i-$C_3H_7$ | $CH_3$ |
| 60 | " | $C_2H_5$ | $C_2H_5$ |
| 61 | " | n-$C_4H_9$ | $CH_3$ |

- to be continued -

Table 1 (continued)

| Compound No. | Ar | $R^1$ | $R^2$ |
|---|---|---|---|
| 62 | 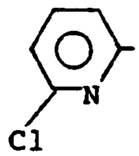 | sec-$C_4H_9$ | $CH_3$ |
| 63 | " | 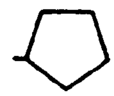 | H |
| 64 | " | 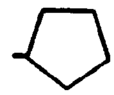 | $CH_3$ |
| 65 | " | 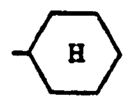 | $CH_3$ |
| 66 | " | 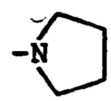 | |
| 67 | " | 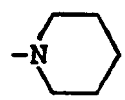 | |
| 68 | " | 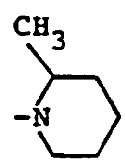 | |
| 69 | " | 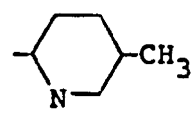 | |
| 70 | " | 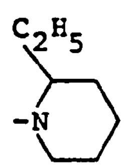 | |

- to be continued -

Table 1 (continued)

| Compound No. | Ar | $R^1$ | $R^2$ |
|---|---|---|---|
| 71 | 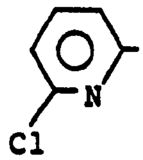 | 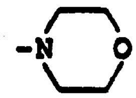 | |
| 72 | " | 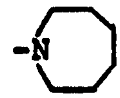 | |
| 73 | 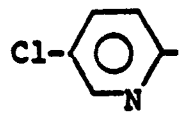 | 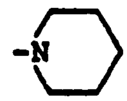 | |
| 74 | " | 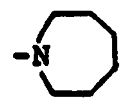 | |
| 75 | 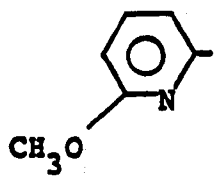 | 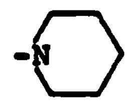 | |
| 76 | 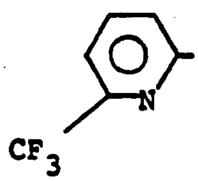 | 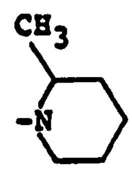 | |
| 77 | " | 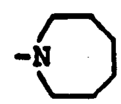 | |
| 78 | 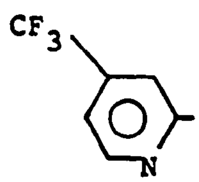 | 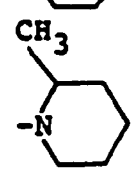 | |
| 79 | " | 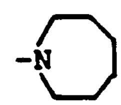 | |
| 80 | 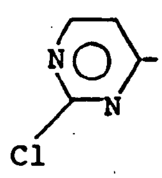 | 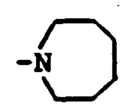 | |

- to be continued -

Table 1 (continued)

| Compound No. | Ar | $R^1$ | $R^2$ |
|---|---|---|---|
| 82 | 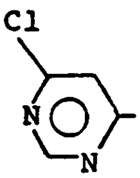 | 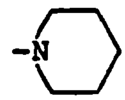 | |
| 83 | 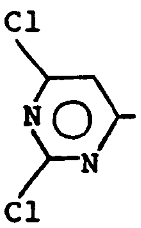 | " | |
| 84 | 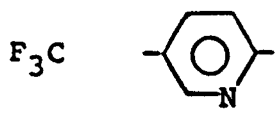 | " | |
| 85 | 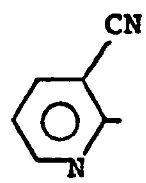 | " | |
| 86 | 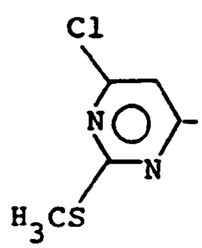 | " | |
| 87 | 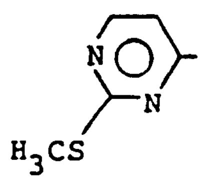 | " | |
| 88 | 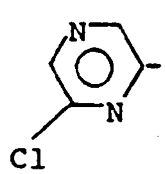 | " | |

- to be continued -

Table 1 (continued)

| Compound No. | Ar | $R^1$ | $R^2$ |
|---|---|---|---|
| 89 | 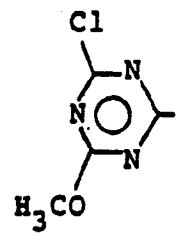 | 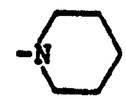 | |
| 90 | 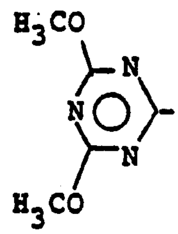 | ″ | |
| 91 | 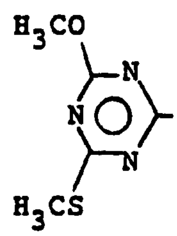 | ″ | |
| 92 | 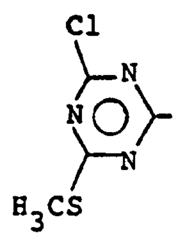 | ″ | |
| 93 | 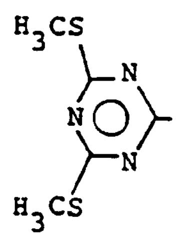 | ″ | |

Among the compounds tabulated in Table 1, compounds Nos. 1-5, 7, 11, 16, 17, 21-26, 30, 31, 35-38, 41, 43-45, 48, 51, 53, 59-62, 64, 65, 67-72, 74, 76, 77, 79, 81, 84, 86-91, and 93 are preferred. Compounds Nos. 2, 3, 7, 11, 21, 22, 43, 44, 53, 60, 62, 64, 65, 68-70, 76, 77, 84, 86-88, and 93 are especially preferred.

The substances in accordance with this invention may be in a free form, or in the form of a salt such as an acid addition salt. Examples of the acid that constitutes the acid addition salt may include, for example, mineral acids such as hydrochloric acid, sulfuric acid and phosphoric acid, and organic acids such as acetic acid, chloroacetic acid, trichloroacetic acid, maleic acid and citric acid.

According to this invention, substituted aryloxyureas of the following formula (I)-1 which are among the compounds of this invention represented by formula (I)

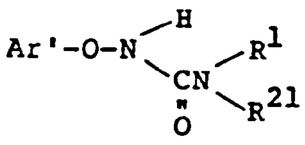

..... (I)-1

wherein Ar' is a group selected from the class consisting of the aryl groups of formulae (a) to (g) defined as Ar in formula (I), $R^1$ is as defined with regard to formula (I), $R^{21}$ represents a $C_1$-$C_6$ alkyl group, and $R^1$ and $R^{21}$ may form the same heterocyclic group as the 4- to 8-membered heterocyclic group formed by $R^1$ and $R^2$ in formula (I),
can be produced by reacting an aryloxyamine represented by the following formula (II)

$$Ar'\text{-}ONH_2 \quad (II)$$

wherein Ar' is as defined above,
with a carbamic acid chloride represented by the following formula (III)

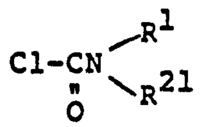

..... (III)

wherein $R^1$ and $R^{21}$ are as defined above with regard to formula (I),
in the presence of a base.

In formula (II), Ar' is a group selected from the class of formulae (a) to (g) in the definition of Ar in formula (I). The definition of $R^{21}$ differs from that of $R^2$ only in that the former does not include a hydrogen atom. $R^1$ is the same as defined with regard to formula (I). Hence, examples of the compound of formula (II) will be apparent to those skilled in the art from the specific examples of the compounds of formula (I).

The compound of formula (II) can be produced by the methods described in European Patent Application EP-183174, Japanese Laid-Open Patent Publication No. 126065/1986, and Japanese Patent Applications Nos. 257691/1985 and 119293/1986 and basically in accordance with these methods.

Specific examples of the compound of formula (III) will also be apparent to those skilled in the art from the definitions of $R^1$ and $R^2$ in formula (I).

The compound of formula (II) and the carbamic acid chloride of formula (III) are reacted in the presence of a base.

The base may be an organic base or an inorganic base. Preferred examples of the organic base are pyridine bases such as pyridine, picoline, lutidine and collidine and tertiary amines such as triethylamine, 1,8-diazabicyclo[5.4.0]undecene-7 and N,N-dimethylaniline.

Examples of the inorganic base are $NaHCO_3$, $KHCO_3$, $Na_2CO_3$ and $K_2CO_3$.

In the reaction, the carbamic acid chloride of formula (III) is used usually in an amount of 0.8 to 3 moles, preferably 1 to 2 moles, per mole of the compound of formula (II). Usually, the base is used in an amount of 0.5 to 20 moles, preferably 1 to 10 moles, per mole of the carbamic acid chloride in formula (III).

The reaction temperature is usually -20 to 100 °C, preferably -10 to 60 °C. The reaction time is usually 30 minutes to 30 hours. Desirably, the reaction is carried out with stirring.

Use of a reaction solvent is not essential. If desired, a solvent inert to the reaction may be used. Examples of such a solvent include aromatic hydrocarbons such as benzene, toluene and xylene, halogenated hydrocarbons such as chloroform, dichloromethane, carbon tetrachloride, dichloroethane, trichloroethane, tetrachloroethane, chlorobenzene and dichlorobenzene, tetrahydrofuran, ethyl acetate and dimethylformamide.

After the reaction, the desired compound may be obtained from the reaction mixture by conventional

methods such as those shown in Examples given hereinafter.

According to this invention, substituted aryloxyureas of the following formula (I)-2 which are within the compounds of formula (I)

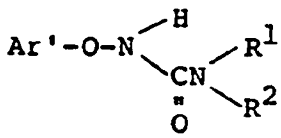

..... (I)-2

wherein Ar′, $R^1$ and $R^2$ are as defined above, can be produced by reacting an aryloxycarbamic acid ester represented by the following formula (IV)

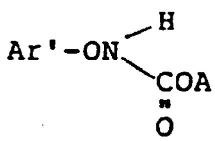

..... (IV)

wherein Ar′ is as defined above, and A represents a substituted or unsubstituted phenyl group,

with an amine represented by the following formula (v)

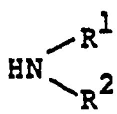

..... (V)

wherein $R^1$ and $R^2$ are as defined above.

In formula (IV), Ar′ is as defined above, and A is a substituted or unsubstituted phenyl group. Examples of preferred substituted phenyl groups are halogenated phenyl groups such as chlorophenyl and lower alkyl-substituted phenyl groups such as tolyl.

The aryloxycarbamic acid ester of formula (IV) can be produced by reacting the aryloxyamine of formula (II) above with a compound represented by AOCOCl in the presence of a base and a solvent. The base and the solvent may be the same as those exemplified above with regard to the process for producing the compounds of formula (I)-1.

In formula (V), the definitions of $R^1$ and $R^2$ are the same as those given above with regard to formula (I).

In the reaction, the amine of formula (V) is used in an amount of usually 0.8 to 5 moles, preferably 1 to 3 moles, per mole of the aryloxycarbamic acid ester of formula (IV).

The reaction temperature is usually 0 to 100 °C, preferably 0 to 80 °C. The reaction time is usually 30 minutes to 5 hours. Desirably, the reaction is carried out with stirring.

Preferably, the reaction is carried out in a solvent. Examples of the solvent may be the same as exemplified above with regard to the process for producing the compounds of formula (I)-1. When an amine of formula (V) in which $R^2$ is a $C_1$-$C_6$ alkyl group is used, it is especially desirable to use dimethylformamide as the reaction solvent.

Furthermore, according to this invention, substituted aryloxyureas represented by the following formula (I)-3 which are among the compounds of formula (I)

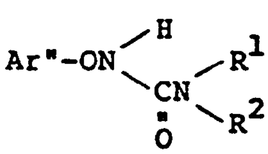

..... (I)-3

wherein Ar″ is a group selected from the class consisting of the aryl groups of formulae (h), (i) and (j) in formula (I), and $R^1$ and $R^2$ are as defined with regard to formula (I),

can be produced by reacting a chloride selected from the group consisting of chlorinated pyrimidines represented by the following formula (VI)

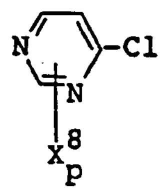

..... (VI)

wherein $X^8$ and p are as defined with regard to formula (I), chlorinated triazines represented by the following formula (VII)

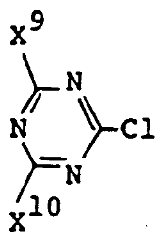

..... (VII)

wherein $X^9$ and $X^{10}$ are as defined with regard to formula (I), and chlorinated pyrazines represented by the following formula (VIII)

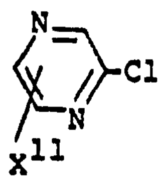

..... (VIII)

wherein $X^{11}$ is as defined with regard to formula (I), with an N-hydroxyurea represented by the following formula (IX)

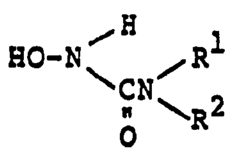

..... (IX)

wherein $R^1$ and $R^2$ are as defined with regard to formula (I), in the presence of a base.

The definitions of $X^8$ and p in formula (VI) are the same as in formula (I), and the definitions of $X^9$ and $X^{10}$ in formula (VII) are the same as in formula (I). $X^{11}$ in formula (VIII) is also as defined above with regard to formula (I).

Hence, specific examples of the chlorinated pyrimidines of formula (VI), the chlorinated triazines of formula (VII) and the chlorinated pyrazines of formula (VIII) will be apparent from the above-given specific examples of $X^8$, p, $X^9$ and $X^{10}$.

In formula (IX), $R^1$ and $R^2$ are as defined with regard to formula (I), and therefore, specific examples of the N-hydroxyureas of formula (IX) will also be apparent from the specific examples of these groups.

In the reaction, the N-hydroxyurea of formula (IX) is used in an amount of usually 0.8 to 5 moles, preferably 1 to 2 moles, per mole of the chloride selected from the chlorinated pyrimidines of formula (VI) and the chlorinated triazines of formula (VII).

The reaction temperature is usually -50 °C to 50 °C, preferably -30 °C to 40 °C. The reaction time is usually 30 minutes to 10 hours.

Desirably, the reaction is carried out with stirring. The base used in the reaction may be the same as those exemplified above with regard to the process for producing the compounds of formula (I)-1. Alkali metal

alkoxides such as sodium ethoxide and potassium t-butoxide may also be used suitably.

The base is used in an amount of usually 0.5 to 10 moles, preferably 1 to 5 moles, per mole of the chloride used.

The reaction can be carried out without a solvent or in a solvent. The solvent used may be the same as those exemplified above with regard to the process for producing the compounds of formula (I)-1.

The substituted aryloxyureas of formula (I) and their acid addition salts provided by this invention exhibit an excellent and characteristic herbicidal efficacy.

Accordingly, the present invention also provides a herbicide comprising the substituted aryloxyurea or its acid addition salt of the invention as a herbicidally effective ingredient.

As a herbicide, the compounds of this invention may be directly used, or as various formulations such as granules, wettable powders, emulsifiable concentrates, dusts and pulverulent agents. The compounds of this invention show better results when used in these formulations. Herbicides in the form of these formulations can be produced from the compounds of this invention and various adjuvants, for example solid carriers such as talc, bentonite, clay, kaolin, diatomaceous earth, white carbon, vermiculite, slaked lime, silica sand, ammonium sulfate and urea, liquid carriers such as alcohols, dioxane, acetone, cyclohexanone, methylnaphthalene, dimethylformamide and dimethyl sulfoxide, emulsifiers and dispersants such as salts of alkylsulfuric esters, alkylarylsulfonate salts, polyoxyethylene glycol ethers, polyoxyethylene alkylaryl ethers and polyoxyethylene sorbitan monoalkylates, and carboxymethyl cellulose and gum arabic. As required, the proportion of the active ingredient may be adjusted. For prepartion of a dust, it is suitably 0.5 to 20 % by weight, and for an emulsifiable concentrate or a wettable powder, it is suitably 5 to 70 % by weight.

The herbicide of this invention may be applied as such or in a form suitably diluted or suspended in water, etc. in an amount effective for controlling weeds. The amount of the compound of this invention used as a herbicide cannot be generalized because it varies depending upon the soil conditions, the formulation, preparation, the time of application, the method of application, the types of the crops or weeds to be controlled, etc. It is effective to apply it in an amount of 10 g to 5 kg per hectare.

The herbicide of this invention exhibits a better herbicidal efficacy than commercial herbicides by application to annual weeds such as barnyardgrass (Echinochloa crus-galli), umbrella plant (Cyperus difformis), monochoria (Monochoria vaginalis), "kikashigusa" (Rotala indica), false pimpernel (Lindernia pyxidaria) and "abunome" (Dopatrium junceum) and perennial weeds such as bulrush (Scirpus juncoides), spikerush (Eleocharis acicularis), "mizugayatsuri" (Cyperus serotinus) and narrowleaf waterplaintain (Alisma canaliculatum), which occur in paddies, during their germination and growth. On the other hand, the herbicide of this invention is not phytotoxic to useful crops, particularly rice, even at high dosages, and has very high selectivity. Furthermore, by soil treatment or foliar treatment, the herbicide of this invention shows a high herbicidal efficacy against various weeds causing hazards in upland farms, for example gramineous weeds such as barnyardgrass, crabgrass (Digitaria sanguinals), green foxtail (Setaria viridis), annual bluegrass (Poa annua) and water foxtail (Alopecurus geniculatus), cyperous weeds such as rice flatsedge (Cyperus iria) and broad-leaved weeds such as redroot pigweed (Amaranthus retroflexus) and lambsquarters (Chenopodium album), and yet show high safety on principal crops such as rice, wheat, corn, soybeans and cotton. The herbicide of this invention can also be used to control weeds in orchards, pasture land, lawns and non-agricultural lands.

If required, the herbicide of this invention may be used in combination with other agricultural chemicals and feeds, for example.

## EXAMPLES

### Example 1A

1,1-Hexamethylene-3-(3-nitrophenoxy)urea (compound No. 1):-

3-Nitrophenoxyamine (3.02 g; 19.6 mmoles) was dissolved in 7.9 ml of pyridine, and 4.75 g (29.4 mmoles) of N,N-hexamethylenecarbamoyl chloride was added. The mixture was stirred at 25 °C for 10 hours and then at 35 to 40 °C for 6.5 hours. A saturated aqueous solution of sodium chloride (150 ml) was added to the reaction mixture, and the mixture was extracted with ethyl acetate. The extract was dried over anhydrous magnesium sulfate, and ethyl acetate was evaporated under reduced pressure. Recrystallization of the residue from toluene/hexane gave 3.92 g (yield 72 %) of the captioned compound as yellow crystals.

Melting point: 97 - 99 °C

Mass spectrum (FD method): m/z 279 (molecular ion peak)

IR spectrum (KBr tablet, $cm^{-1}$): 3130, 1643, 530, 1474, 1347, 736

$^{1}H$-NMR spectrum ($CDCl_3$ solution, ppm)

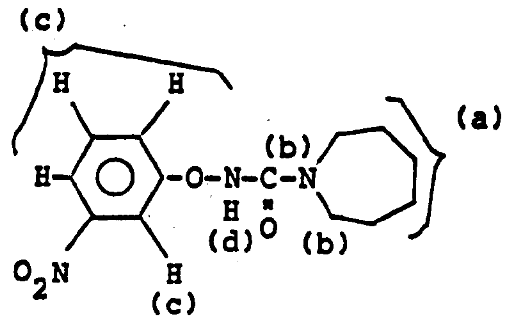

(a) 1.5 - 2.0(8H, m)

(b) 3.44(4H, m)

(c) 7.2 - 7.9(4H, m) (d) 7.96(1H, s)

Example 2A

1-Cyclopentyl-3-(5-methyl-2-nitrophenoxy)urea (compound No. 2):-

Phenyl N-(5-methyl-2-nitrophenoxy)carbamate (3.86 g, 13.4 mmoles) was dissolved in 60 ml of ethyl acetate, and 2.85 g (33.5 mmoles) of cyclopentylamine was added. The mixture was stirred at 60 °C for 1.5 hours. The reaction mixture was cooled to room temperature, washed with a 3% aqueous solution of sodium hydroxide and then with a saturated sodium chloride solution, and dried over anhydrous magnesium sulfate. Ethyl acetate was evaporated under reduced pressure, and the residue was recrystallized from toluene-hexane to give 1.83 g (yield 49 %) of the captioned compound as yellow crystals.

Melting point: 131.- 132 °C (decomp.)

Mass spectrum (FD method): m/z 279 (molecular ion peak)

IR spectrum (KBr tablet, $cm^{-1}$): 3390, 1660, 1605, 1590, 1340, 1310, 1260, 1240

$^1$H-NMR spectrum ($CDCl_3$ solution, ppm)

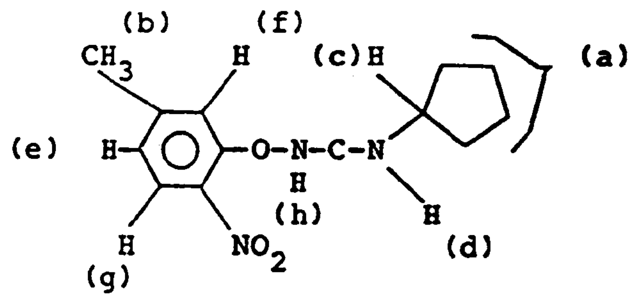

(a) 1.4-2.2(8H, m)

(b) 2.44(3H, s)

(c) 4.16(1H, m)

(d) 6.74(1H, d, J=7.2Hz)

(e) 6.98(1H, dd, J=8.3, 1.3Hz)

(f) 7.48(1H, d, J=1.3Hz)

(g) 7.82(1H, d, J=8.3Hz)

(h) 8.7(1H, br.s)

Example 3A

3-(3-Cyanophenoxy)-1,1-hexamethyleneurea (compound No. 3):-

Phenyl N-(3-cyanophenoxy)carbamate (2.10 g; 8.3 mmoles) was dissolved in a mixed solvent of 20 ml of N,N-dimethylformamide and 20 ml of toluene. Then, 1.64 g (16.5 mmoles) of hexamethylenimine was added. The mixture was stirred at 60 °C for 1 hour. The reaction mixture was cooled to room temperature, and after adding 120 ml of water, extracted with ethyl acetate. The exract was washed with a saturated aqueous sodium chloride solution, and dried over anhydrous magnesium sulfate. Ethyl acetate was evaporated under reduced pressure. The residue was purified by silica gel column chromatography (eluting solvent: ethyl acetate/hexane). Recrystallization from toluene/hexane gave 2.05 g (yield 91 %) of the captioned compound as pale yellow crystals.

Melting point: 77 - 79 °C.

Mass spectrum (FD method): m/z 259 (molecular ion peak)

IR spectrum (KBr tablet, $cm^{-1}$) 3285, 2032, 1656, 1575, 1482, 1405, 1241, 1205, 800, 684

$^1$H-NMR spectrum ($CDCl_3$ solution, ppm)

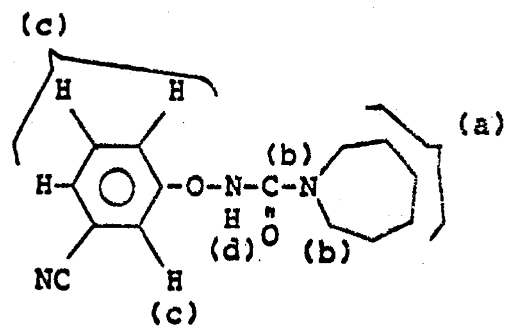

(a) 1.4-2.0(8H, m)

(b) 3.40(4H, m)

(c) 7.36(4H, m)

(d) 8.04 (1H, s)

Example 4A

3-(2-Chloro-4-pyrimidinyl)-1,1-pentamethyleneurea (compound No. 4):-

2,4-Dichloropyrimidine (3.00 g; 20.1 mmoles) and 2.90 g (20.1 mmoles) of 3-hydroxy-1,1-pentamethyleneurea were dissolved in 40 ml of-N,N-dimethylformamide (DMF). The solution was cooled to -25 °C, and 20 ml of a DMF solution of 2.26 g (20.1 mmoles) of potassium t-butoxide was added dropwise over the course of 25 minutes. The mixture was stirred at -25 to -10 °C for 3 hours and then 250 ml of water was added. The mixture was extracted with ethyl acetate. The extract was washed with a saturated aqueous solution of sodium chloride, and dried over anhydrous magnesium sulfate. Ethyl acetate was evaporated under reduced pressure, and the residue was purified by silcia gel column chromatography (eluting solvent: ethyl acetate/hexane). Recrystallization from toluene/hexane gave 0.78 g (yield 15 %) of the captioned compound as colorless crystals.

Melting point: 136 - 138 °C

Mass spectrum (FD method): m/z 259 (molecular ion peak)

IR spectrum (KBr tablet, $cm^{-1}$) 3200, 1660, 1555, 1510, 1408, 1347, 1294, 1265, 1252, 1220, 1210, 1192, 1076, 1015, 978, 910, 862, 840, 765, 720

$^1$H-NMR spectrum ($CDCl_3$ solution, ppm)

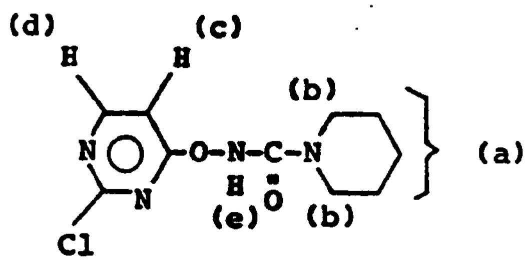

(a) 1.65(6H, m)

(b) 3.44(4h, m)

(c) 6.96(1H, d, J=5.7Hz)

(d) 8.42(1H, d, J=5.7Hz)

(e) 8.78(1H, s)

Examples 5A-80A

In the same way as in Examples 1A to 4A, compounds Nos. 5 to 80 shown in Table 1 were synthesized from the corresponding starting materials. The results are shown in Table 1A. Methods A, B, C and D in Table 1A show that they correspond respectively to the methods described in Examples 1A, 2A, 3A and 4A.

**Table 1A**

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 5 | A | 81 | 113 - 115 (decomp.) | 1645 | 3200 | |
| 6 | B | 59 | 103 - 105 (decomp.) | 1650 | 3500, 3170 | |
| 7 | A | 52 | 112 - 114 (decomp.) | 1643 | 3200 | |
| 8 | B | 75 | 120 - 121 (decomp.) | 1660 | 3390, 3150 | |
| 9 | A | 42 | 109 - 110 (decomp.) | 1650 | 3120 | |
| 10 | B | 85 | 139 - 140.5 (decomp.) | 1665 | 3400, 3160 | |
| 11 | A | 65 | 118 - 120 (decomp.) | 1655 | 3200 | |
| 12 | A | 32 | 116 - 120 | 1660 | 3140 | |

- to be continued -

**Table 1A (continued)**

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 13 | A | 64 | 121 - 123 (decomp.) | 1665 | 3050 | |
| 14 | B | 66 | 120 - 120.5 (decomp.) | 1655 | 3290, 3250 | |
| 15 | B | 85 | 136 - 137 (decomp.) | 1660 | 3290, 3260 | |
| 16 | A | 40 | 105 - 106 (decomp.) | 1640 | 3100 | |
| 17 | A | 48 | 105 - 106.5 (decomp.) | 1650 | 3100 | |
| 18 | A | 14 | 142 - 144 (decomp.) | 1660 | 3095 | |
| 19 | A | 68 | 160 - 161 (decomp.) | 1660 | 3090 | |
| 20 | A | 39 | 118 - 120 (decomp.) | 1660 | 3160 | |

- to be continued -

Table 1A (continued)

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 21 | B | 75 | 122 - 125 (decomp.) | 1650 | 3340 | |
| 22 | B | 77 | 138 - 140 (decomp.) | 1660 | 3290 | |
| 23 | C | 87 | 123 - 125 | 1655 | 3275 | |
| 24 | A | 28 | 127.5 - 128.5 (decomp.) | 1670 | 3140 | 1510, 1340($\nu_{NO_2}$) |
| 25 | B | 32 | 112 - 114 | 1670 | 3395, 3150, 3080 | 1520, 1350($\nu_{NO_2}$) |
| 26 | A | 52 | 122 - 124 (decomp.) | 1665 | 3190 | 3290($\nu_{\equiv C-H}$), 1515, 1355($\nu_{NO_2}$) |
| 27 | A | 70 | 132 - 133.5 | 1670 | 3190 | 1520, 1350($\nu_{NO_2}$) |
| 28 | A | 78 | 80 - 82 | 1655 | 3150 | 1640($\nu_{C=C}$), 1530, 1355($\nu_{NO_2}$) |

- to be continued -

Table 1A (continued)

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 29 | A | 71 | 145 - 147 | 1660 | 3180, 3110 | 1525, 1355($\nu_{NO_2}$) |
| 30 | A | 67 | 102.5 - 104.5 | 1645 | 3110 | 1540, 1355($\nu_{NO_2}$) |
| 31 | A | 46 | 150 - 152 (decomp.) | 1670 | 3200 | 1540, 1340($\nu_{NO_2}$) |
| 32 | B | 79 | 143 - 144.5 (decomp.) | 1660 | 3400, 3350, 3250 | 1540, 1340($\nu_{NO_2}$) |
| 33 | B | 62 | 136 - 138 (decomp.) | 1670 | 3420, 3140, 3090 | 1545, 1330($\nu_{NO_2}$) |
| 34 | B | 68 | 136 - 138 (decomp.) | 1670 | 3380, 3140, 3090 | 1530, 1345($\nu_{NO_2}$) |
| 35 | B | 63 | 136 - 137 | 1665 | 3320, 3220 | 1640($\nu_{C=C}$), 1510, 1345($\nu_{NO_2}$) |
| 36 | B | 80 | 139 - 141 | 1660 | 3380, 3150 | 1530, 1345($\nu_{NO_2}$) |

- to be continued -

Table 1A (continued)

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 37 | B | 32 | 142 - 144 | 1675 | 3380, 3160 | 1540, 1335($\nu_{NO_2}$) |
| 38 | B | 53 | 146 - 148 | 1685 | 3380, 3160 | 3300($\nu_{\equiv C-H}$) |
| 39 | B | 74 | 153 - 155 (decomp.) | 1665 | 3390, 3150 | 1535, 1335($\nu_{NO_2}$) |
| 40 | B | 47 | 166 - 168 (decomp.) | 1665 | 3400, 3100 | 1530, 1330($\nu_{NO_2}$) |
| 41 | A | 49 | 112 - 113 | 1665 | 3100 | 1510, 1345($\nu_{NO_2}$) |
| 42 | B | 31 | 158 - 160 (decomp.) | 1655 | 3380, 3140, 3090 | 1535, 1340($\nu_{NO_2}$) |
| 43 | A | 18 | 121 - 123 | 1655 | 3110 | 1515, 1345($\nu_{NO_2}$) |
| 44 | A | 9 | 132 - 134 | 1665 | 3110 | 1515, 1345($\nu_{NO_2}$) |
| 45 | A | 45 | 139 - 141 (decomp.) | 1665 | 3160 | 1510, 1340($\nu_{NO_2}$) |

- to be continued -

Table 1A (continued)

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 46 | B | 58 | 157 - 159 (decomp.) | 1670 | 3410, 3170 | 1510, 1340($\nu_{NO_2}$) |
| 47 | A | 20 | 116.5 - 118.5 (decomp.) | 1655 | 3120 | 1510, 1335($\nu_{NO_2}$) |
| 48 | B | 24 | 114 - 116 | 1665 | 32710 | 1530, 1320($\nu_{NO_2}$) |
| 49 | B | 22 | 126 - 128 | 1670 | 3300, 3230 | 1530, 1320($\nu_{NO_2}$) |
| 50 | C | 59 | 115 - 117 | 1650 | 3270 | |
| 51 | C | 65 | 137 - 139 | 1655 | 3220 | 2230($\nu_{C\equiv N}$) |
| 52 | A | 79 | 151 - 152 (decomp.) | 1670 | 3130 | 2220($\nu_{C\equiv N}$) |
| 53 | C | 71 | 140.5 - 142 | 1665 | 3200 | 2240($\nu_{C\equiv N}$) |
| 54 | C | 6 | 153 - 155 | 1645 | 3220 | |
| 55 | B | 52 | 163 - 165 (decomp.) | 1650 | 3300, 3250 | 2220($\nu_{C\equiv N}$) |

- to be continued -

Table 1A (continued)

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 56 | C | 30 | 99 - 101 | 1650 | 3260 | |
| 57 | C | 27 | liquid | 1675 | 3190 | |
| 58 | B | 70 | 103 - 105 | 1660 | 3300, 3160 | |
| 59 | A | 55 | 121 - 122 | 1645 | 3180 | |
| 60 | C | 35 | 64 - 66 | 1660 | 3110 | |
| 61 | C | 63 | 76 - 78 | 1660 | 3140 | |
| 62 | A | 55 | 102 - 104 | 1650 | 3185 | |
| 63 | B | 88 | 141 - 143 | 1660 | 3280, 3170, 3080 | |
| 64 | A | 65 | 138 - 139 | 1645 | 3180 | |
| 65 | A | 57 | 160 - 163 | 1655 | 3190 | |
| 66 | C | 49 | 161 - 163 | 1665 | 3130 | |
| 67 | C | 56 | 127 - 129 | 1675, 1650 | 3150 | |

- to be continued -

Table 1A (continued)

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 68 | A | 65 | 109 - 111 | 1640 | 3150 | |
| 69 | C | 42 | 121 - 123 | 1665 | 3180 | |
| 70 | A | 56 | 99 - 101 | 1655 | 3200 | |
| 71 | C | 53 | 131 - 132 | 1665 | 3205 | |
| 72 | A | 32 | 129 - 131 | 1655 | 3200, 3160 | |
| 73 | C | 55 | 114 - 116 | 1640 | 3160 | |
| 74 | C | 49 | 104 - 106 | 1650 | 3030 | |
| 75 | C | 71 | 94 - 96 | 1650 | 3220 | |
| 76 | A | 29 | 130 - 132 | 1640 | 3190 | |
| 77 | A | 29 | 146 - 148 | 1655 | 3200, 3160 | |
| 78 | A | 26 | 92 - 94 | 1645 | 3230 | |
| 79 | A | 15 | 101 - 103 | 1655, 1645 | 3220 | |
| 80 | D | 24 | 132 - 134 (decomp.) | 1665 | 3180, 3150 | |

Table 1A (continued)

| Compound No. | Method | Yield (%) | Melting point (°C) | IR spectrum ($cm^{-1}$) | | |
|---|---|---|---|---|---|---|
| | | | | $\nu_{C=O}$ | $\nu_{N-H}$ | Other characteristic absorption |
| 82 | D | 24 | 102 - 104 | 1655 | 3210 | |
| 83 | D | 20 | 86 - 88 | 1660 | 3150 | |
| 84 | C | 20 | 120 - 123 | 1655 | 3150 | |
| 85 | C | 39 | 236 - 239 | 1665 | 3180 | |
| 86 | D | 31 | 102 - 104 | 1655 | 3150 | |
| 87 | D | 20 | liquid | 1660 | 3180 | |
| 88 | D | 24 | 127 - 129 | 1655 | 3200 | |
| 89 | D | 20 | 125 - 127 | 1660 | 3150 | |
| 90 | D | 33 | 114 - 116 | 1655 | 3180 | |
| 91 | D | 56 | 135 - 137 | 1660 | 3150 | |
| 92 | D | 19 | 123 - 125 | 1660 | 3140 | |
| 93 | D | 51 | 169 - 171 (decomp.) | 1660 | 3130 | |

Examples of formulating the herbicide of this invention will now be given. In the following Formulation Examples, all percentages are by weight.

Formulation Example 1B (granules)

The compound of this invention (10 %), 2 % of a sodium salt of lauryl sulfate, 5 % of sodium ligninsulfonate, 2 % of carboxymethyl cellulose and 81 % of clay were uniformly mixed and pulverized.

Water (20 parts) was added to 80 parts of the resulting mixture and they were kneaded. The kneaded mixture was molded into particles having a size of 14 to 32 mesh by an extrusion granulator, and then dried to form granules.

Formulation Example 2B (dust)

A sodium salt of lauryl sulfate (2 %), 5 % of sodium ligninsulfonate, 2 % of carboxymethyl cellulose and 91 % of a clay/montmorillonite mixture were uniformly mixed and pulverized. The mixture (78 parts) was kneaded with 22 parts of water, and the kneaded mixture was processed into particles having a size of 14 to 32 mesh by an extrusion granulator and then dried to form a base composition for adsorption. Twenty parts of a solution of 20 % of the compound of this invention in 80 % of polyethylene glycol was adsorbed uniformly on 80 parts of the base composition to form a dust.

Formulation Example 3B (wettable powder)

The compound of the invention (10 %), 85 % of diatomaceous earth, 2 % of sodium dinaphthylmethanedisulfonate and 3 % of sodium ligninsulfonate were uniformly mixed and pulverized to form a wettable powder.

Fomulation Example 4B (emulsificable concentrate)

The compound of this invention (30 %), 20 % of cyclohexanone, 11 % of polyoxyethylene alkylaryl ether, 4 % of calcium alkylbenzenesulfonate and 35 % of methylnaphthalene were dissolved uniformly to form an emulsifiable concentrate.

Formulation Example 5B (dust)

The compound of this invention (4 %), 5 % of diatomaceous earth and 91 % of clay were uniformly mixed and pulverized to form a dust.

The efficacy of the herbicide of this invention will now be illustrated by the following Test Examples.

Test Example 1C (herbicidal test by soil treatment in a paddy)

Porcelain pots, 10 cm in diameter, were filled with paddy soil, and after puddling, seeds of barnyardgrass, umbrella plant, monochoria and bulrush were sown. The pots were then watered to a depth of 3 cm. On the next day, a wettable powder prepared in accordance with Formulation Example 3B was diluted with water and dropped onto the water surface (amount applied: 4 kg per hectare as the active ingredient). The plants were then grown in a greenhouse, and 30 days after the treatment, the herbicidal activity of the compound of the invention was examined according to the standards given in Table 1C. The results are shown in Table 2C.

## Table 1C

| Index | Herbicidal efficacy and phytotoxicity |
|---|---|
| 5 | Withered |
| 4.5 | Herbicidal efficacy of 90 to 99 % (phytotoxic) |
| 4 | Herbicidal efficacy of 80 to 89 % (phytotoxic) |
| 3.5 | Herbicidal efficacy of 70 to 79 % (phytotoxic) |
| 3 | Herbicidal efficacy of 60 to 69 % (phytotoxic) |
| 2.5 | Herbicidal efficacy of 50 to 59 % (phytotoxic) |
| 2 | Herbicidal efficacy of 40 to 49 % (phytotoxic) |
| 1.5 | Herbicidal efficacy of 30 to 39 % (phytotoxic) |
| 1 | Herbicidal efficacy of 20 to 29 % (phytotoxic) |
| 0.5 | Herbicidal efficacy of 19 to 1% (phytotoxic) |
| 0 | No herbicidal efficacy (no phytotoxicity) |

Table 2C

| Test compound No. | Herbicidal effect | | | |
|---|---|---|---|---|
| | barnyard-grass | umbrella plant | monochoria | bulrush |
| 1 | 5 | 5 | 5 | 5 |
| 2 | 5 | 5 | 5 | 5 |
| 3 | 5 | 5 | 5 | 5 |
| 6 | 5 | 5 | 5 | 5 |
| 7 | 5 | 5 | 5 | 5 |
| 8 | 5 | 5 | 5 | 5 |
| 9 | 4 | 4 | 5 | 4 |
| 10 | 5 | 5 | 5 | 5 |
| 14 | 5 | 5 | 5 | 5 |
| 15 | 5 | 5 | 5 | 5 |
| 16 | 5 | 5 | 5 | 5 |
| 17 | 5 | 5 | 5 | 5 |
| 18 | 5 | 5 | 5 | 5 |
| 19 | 5 | 5 | 5 | 5 |
| 21 | 5 | 5 | 5 | 5 |
| 22 | 5 | 5 | 5 | 5 |
| 23 | 5 | 5 | 5 | 5 |
| 24 | 5 | 5 | 4 | 5 |
| 25 | 5 | 5 | 5 | 5 |
| 26 | 5 | 5 | 5 | 5 |

- to be continued -

Table 2C (continued)

| Test compound No. | Herbicidal effect | | | |
|---|---|---|---|---|
| | barnyard-grass | umbrella plant | monochoria | bulrush |
| 28 | 5 | 5 | 5 | 5 |
| 30 | 5 | 5 | 5 | 5 |
| 32 | 5 | 5 | 5 | 5 |
| 33 | 5 | 5 | 5 | 5 |
| 34 | 5 | 5 | 5 | 5 |
| 35 | 5 | 5 | 5 | 5 |
| 36 | 5 | 5 | 5 | 5 |
| 37 | 5 | 5 | 5 | 5 |
| 38 | 5 | 5 | 5 | 5 |
| 39 | 5 | 5 | 5 | 5 |
| 40 | 5 | 5 | 5 | 5 |
| 41 | 5 | 5 | 5 | 5 |
| 42 | 5 | 5 | 5 | 5 |
| 43 | 5 | 5 | 5 | 5 |
| 44 | 5 | 5 | 5 | 5 |
| 45 | 5 | 4 | 2 | 4 |
| 46 | 5 | 5 | 5 | 3 |
| 48 | 5 | 5 | 5 | 5 |
| 49 | 5 | 5 | 5 | 4 |
| 51 | 5 | 5 | 5 | 5 |

- to be continued -

Table 2C (continued)

| Test compound No. | Herbicidal effect | | | |
|---|---|---|---|---|
| | barnyard-grass | umbrella plant | monochoria | bulrush |
| 53 | 5 | 5 | 5 | 5 |
| 55 | 5 | 5 | 5 | 5 |
| 56 | 5 | 5 | 4 | 5 |
| 59 | 5 | 5 | 5 | 5 |
| 60 | 5 | 5 | 5 | 5 |
| 61 | 5 | 5 | 5 | 5 |
| 62 | 5 | 5 | 5 | 5 |
| 63 | 5 | 5 | 4 | 5 |
| 64 | 5 | 5 | 5 | 5 |
| 65 | 5 | 5 | 5 | 5 |
| 66 | 5 | 5 | 5 | 5 |
| 67 | 5 | 5 | 5 | 5 |
| 68 | 5 | 5 | 5 | 5 |
| 69 | 5 | 5 | 5 | 5 |
| 70 | 5 | 5 | 5 | 5 |
| 71 | 5 | 5 | 5 | 4 |
| 72 | 5 | 5 | 5 | 5 |
| 73 | 5 | 5 | 5 | 5 |
| 74 | 5 | 5 | 5 | 5 |
| 75 | 5 | 5 | 5 | 5 |

- to be continued -

Table 2C (continued)

| Test compound No. | Herbicidal effect | | | |
|---|---|---|---|---|
| | barnyard-grass | umbrella plant | monochoria | bulrush |
| 76 | 5 | 5 | 5 | 5 |
| 77 | 5 | 5 | 5 | 5 |
| 78 | 5 | 5 | 3 | 5 |
| 79 | 5 | 5 | 5 | 5 |
| Comparison* | 0 | 0 | 0 | 0 |
| Comparison** | 2 | 1 | 2 | 0 |

*

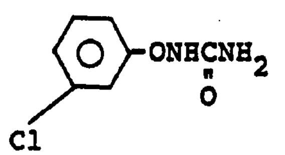

(the compound described in U.S. 3,332,975)

**

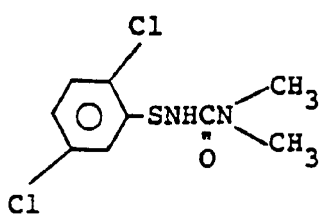

(mp. 143-144°C)

Test Example 2C (selective herbicidal test between rice and barnyardgrass)

Paddy soil was filled into Wagner pots (1/5,000 a), and after puddling, seeds of barnyardgrass were sown and grown to the 2-leaf stage in a greenhouse. The number of barnyardgrass plants in the two-leaf stage per pot was adjusted to 15. A predetermined amount of a wettable powder of each of the test compounds, prepared in accordance with Formulation Example 3B, was diluted with water, and dropped onto the water surface. Separately, rice seedlings in the two-leaf stage were transplanted in other Wagner pots (1/5,000 a). On the next day to the day of transplantation, the test compound was similarly dropped onto the water surface. After the treatment, the rice seedlings were grown for 30 days in a greenhouse. The herbicidal activity and phytotoxicity of the test compound were examined in accordance with the standards given in Table 1C. The results are shown in Table 3C.

Table 3C

| Test compound No. | Amount applied (kg/ha) | Herbicidal efficacy | Phytotoxicity |
|---|---|---|---|
| | | barnyardgrass in the 2-leaf stage | rice |
| 1 | 0.5<br>0.25<br>0.125 | 5<br>5<br>4 | 0<br>0<br>0 |
| 7 | 0.5<br>0.25<br>0.125 | 5<br>5<br>5 | 1<br>0<br>0 |
| 30 | 1<br>0.5<br>0.25 | 5<br>5<br>5 | 1<br>0<br>0 |
| 37 | 1<br>0.5<br>0.25 | 5<br>4.5<br>4 | 1<br>0<br>0 |
| 40 | 1<br>0.5<br>0.25 | 5<br>5<br>4.5 | 0<br>0<br>0 |
| 42 | 1<br>0.5<br>0.25 | 5<br>4.5<br>2.5 | 0<br>0<br>0 |
| 67 | 0.5<br>0.25<br>0.125 | 5<br>5<br>3.5 | 0<br>0<br>0 |

Test Example 3C (herbicidal test by soil treatment in an upland farm)

Upland farm soil was filled in plastic pots (120 $cm^2$), and seeds of barnyardgrass, crabgrass, redroot pigweed and rice flatsedge were sown and covered with the soil. Each of the test compounds was formulated into a wettable powder in accordance with Formulation Example 3B. A predetermined amount of the wettable powder was diluted with water, and using a small-sized sprayer, was uniformly sprayed onto the soil surface at a rate of 1000 liters/hectare (the amount applied: 4 kg per hectare as the active ingredient). The plants were grown for 20 days in a greenhouse after the treatment, and the herbicidal efficacy and phytotoxicity were examined

in accordance with the standards given in Table 1C. The results are shown in Table 4C.

Table 4C

| Test compound No. | Herbicidal efficacy | | | |
|---|---|---|---|---|
| | barnyard-grass | crab-grass | redroot pigweed | rice flats-edge |
| 1 | 5 | 5 | 5 | 5 |
| 2 | 5 | 5 | 1 | 5 |
| 3 | 5 | 5 | 5 | 5 |
| 5 | 5 | 5 | 5 | 5 |
| 6 | 5 | 5 | 4 | 5 |
| 7 | 5 | 5 | 5 | 5 |
| 8 | 4 | 3 | 1 | 5 |
| 9 | 5 | 5 | 5 | 5 |
| 10 | 5 | 5 | 5 | 5 |
| 11 | 5 | 5 | 3 | 5 |
| 13 | 5 | 5 | 3 | 5 |
| 14 | 5 | 5 | 4 | 5 |
| 15 | 4 | 5 | 2.5 | 5 |
| 16 | 5 | 5 | 4 | 5 |
| 17 | 5 | 5 | 3 | 5 |
| 18 | 5 | 4 | 0 | 5 |
| 19 | 4 | 5 | 2 | 5 |

- to be continued -

Table 4C (continued)

| Test compound No. | Herbicidal efficacy | | | |
|---|---|---|---|---|
| | barnyard-grass | crab-grass | redroot pigweed | rice flats-edge |
| 21 | 5 | 5 | 5 | 5 |
| 22 | 5 | 5 | 5 | 5 |
| 23 | 5 | 5 | 5 | 5 |
| 24 | 5 | 5 | 2 | 5 |
| 25 | 5 | 5 | 4 | 5 |
| 26 | 5 | 5 | 4 | 5 |
| 28 | 4 | 5 | 2 | 5 |
| 29 | 3 | 3 | 0 | 5 |
| 30 | 5 | 5 | 5 | 5 |
| 33 | 4 | 5 | 1 | 5 |
| 34 | 4 | 4 | 1 | 5 |
| 35 | 5 | 5 | 1 | 5 |
| 36 | 5 | 5 | 2 | 5 |
| 37 | 5 | 5 | 1 | 5 |
| 38 | 5 | 5 | 2 | 5 |
| 39 | 5 | 4 | 1 | 5 |
| 41 | 5 | 5 | 5 | 5 |
| 43 | 5 | 5 | 4 | 5 |
| 44 | 5 | 5 | 5 | 5 |

- to be continued -

**Table 4C (continued)**

| Test compound No. | Herbicidal efficacy | | | |
|---|---|---|---|---|
| | barnyard-grass | crab-grass | redroot pigweed | rice flats-edge |
| 45 | 4 | 4 | 1 | 5 |
| 47 | 5 | 5 | 3 | 5 |
| 48 | 5 | 4 | 2 | 5 |
| 51 | 5 | 5 | 5 | 5 |
| 52 | 3 | 4 | 3 | 5 |
| 53 | 5 | 5 | 5 | 5 |
| 55 | 5 | 5 | 5 | 5 |
| 59 | 5 | 5 | 5 | 5 |
| 67 | 5 | 5 | 5 | 5 |
| 68 | 5 | 5 | 5 | 5 |
| 70 | 5 | 5 | 5 | 5 |
| 72 | 5 | 5 | 5 | 5 |
| 76 | 5 | 5 | 5 | 5 |
| 77 | 5 | 5 | 5 | 5 |
| 78 | 5 | 4 | 4 | 5 |
| 79 | 5 | 5 | 5 | 5 |

Test Example 4C (herbicidal efficacy and phytotoxicity by soil treatment in an upland farm

Upland farm soil was filled in plastic vats (600 $cm^2$). Seeds of barnyardgrass, crabgrass, green foxtail, redroot pigweed, rice flatsedge, rice, wheat, corn, soybeans, cotton and sugar beet were sown and covered with the soil. Each of the test compounds was formulated into a wettable powder in accordance with Formulation Example 3B. A predetermined amount of the wettable powder was diluted with water and uniformly sprayed

onto the soil surface by a small-sized sprayer at a rate of 1000 liters per hectare. The plants were grown in a greenhouse for 30 days after the treatment. The herbicidal efficacy and phytotoxicity were examined in accordance with the standards given in Table 1C. The results are shown in Table 5C.

Table 5C

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flats-edge | rice | wheat | corn | soy beans | cotton | sugar beet |
| 2 | 2 | 5 | 5 | 5 | 1 | 5 | 0 | 0 | 0 | 0 | 0 | 0 |
| | 1 | 5 | 5 | 5 | 0 | 5 | 0 | 0 | 0 | 0 | 0 | 0 |
| | 0.5 | 4.5 | 5 | 4 | 0 | 5 | 0 | 0 | 0 | 0 | 0 | 0 |
| 7 | 2 | 5 | 5 | 5 | 5 | 5 | 2 | 0.5 | 4 | 1 | 0 | 2 |
| | 1 | 5 | 5 | 5 | 5 | 5 | 2 | 0.5 | 4 | 0 | 0 | 1 |
| | 0.5 | 5 | 5 | 5 | 4 | 5 | 0.5 | 0 | 2 | 0 | 0 | 1 |
| 21 | 2 | 5 | 5 | 5 | 5. | 5 | 4.5 | 2.5 | 3.5 | 0 | 0 | 2 |
| | 1 | 5 | 5 | 5 | 5 | 5 | 2 | 2 | 1 | 0 | 0 | 0 |
| | 0.5 | 4.5 | 5 | 5 | 4 | 5 | 1 | 1 | 0 | 0 | 0 | 0 |
| 22 | 2 | 5 | 5 | 5 | 5 | 5 | 2 | 0.5 | 2.5 | 0 | 0 | 0 |
| | 1 | 5 | 5 | 5 | 5 | 5 | 1 | 0 | 2 | 0 | 0 | 0 |
| | 0.5 | 4.5 | 5 | 5 | 2 | 5 | 0 | 0 | 0.5 | 0 | 0 | 0 |
| 43 | 2 | 5 | 5 | 5 | 5 | 5 | 3 | 1.5 | 3 | 0 | 0 | 0 |
| | 1 | 5 | 5 | 5 | 5 | 5 | 1 | 0 | 3 | 0 | 0 | 0 |
| | 0.5 | 5 | 5 | 4 | 5 | 5 | 0.5 | 0 | 0 | 0 | 0 | 0 |

Test Example 5C (herbicidal test by foliar treatment in an upland farm

Upland farm soil was filled in plastic pots (120 $cm^2$), and seeds of barnyardgrass, crabgrass, redroot pigweed and rice flatsedge were sown, and grown in a greenhouse until the barnyardgrass reached a three-leaf stage. Each of the test compounds was formulated into a wettable powder in accordance with Formulation Example 3B. The wettale powder in an amount equivalent to 4 kg per hectare as the active ingredient was diluted with water, and sprayed over the foliage of the plants from above by a small-sized sprayer at a rate of 1000 liters per hectare. After the spraying, the plants were grown in a greenhouse for 20 days. The herbicidal efficacy was examined in accordance with the standards given in Table 1C, and the results are shown in Table 6C.

Table 6C

| Test compound No. | Herbicidal efficacy | | | |
|---|---|---|---|---|
| | barnyard-grass | crab-grass | redroot pigweed | rice flats-edge |
| 1 | 4 | 5 | 0 | 4 |
| 2 | 4 | 5 | 2 | 5 |
| 3 | 5 | 5 | 3 | 5 |
| 7 | 5 | 5 | 2 | 3 |
| 9 | 5 | 5 | 4 | 5 |
| 11 | 4 | 3 | 1 | 5 |
| 17 | 4 | 4 | 4 | 5 |
| 18 | 3 | 3 | 3 | 5 |
| 19 | 4 | 4 | 5 | 5 |
| 21 | 5 | 5 | 4 | 5 |
| 22 | 5 | 5 | 3 | 5 |
| 23 | 5 | 5 | 4 | 5 |
| 24 | 4 | 4 | 4 | 5 |
| 30 | 4 | 4 | 1 | 4 |
| 35 | 4 | 3 | 0 | 5 |

- to be continued -

Table 6C (continued)

| Test compound No. | Herbicidal efficacy | | | |
|---|---|---|---|---|
| | barnyard-grass | crab-grass | redroot pigweed | rice flats-edge |
| 36 | 4 | 3 | 2 | 4 |
| 37 | 4 | 2 | 1 | 4 |
| 41 | 4 | 4 | 2 | 5 |
| 43 | 4 | 4 | 2 | 5 |
| 44 | 5 | 5 | 4 | 5 |
| 47 | 3 | 3 | 5 | 5 |
| 59 | 5 | 4 | 2 | 5 |
| 67 | 5 | 4 | 3 | 4 |
| 68 | 5 | 4 | 4 | 4 |
| 70 | 5 | 5 | 2 | 5 |
| 72 | 5 | 4 | 3 | 5 |
| 74 | 5 | 4 | 2 | 5 |
| 76 | 5 | 4 | 3 | 5 |
| 77 | 4 | 4 | 2 | 4 |
| 79 | 5 | 4 | 1 | 5 |

Test Example 6C (herbicidal test by soil treatment in a paddy)

Porcelain pots, 10 cm in diameter, were filled with paddy soil, and after puddling, seeds of barnyardgrass, umbrella plant, monochoria and bulrush were sown. The pots were then watered to a depth of 3 cm. On the next day, a wettable powder prepared in accordance with Formulation Example 3B was diluted with water and dropped onto the water surface (amount applied: 4 kg per hectare as the active ingredient). The plants were then grown in a greenhouse, and 30 days after the treatment, the herbicidal activity of the compound of the

invention was examined according to the standards given in Table 1C. The results are shown in Table 7C.

Table 7C

| Test compound No. | Herbicidal efficacy | | | |
|---|---|---|---|---|
| | barn-yard-grass | umbrella plant | monochoria | bulrush |
| 4 | 5 | 5 | 5 | 5 |
| 82 | 5 | 5 | 5 | 5 |
| 83 | 5 | 5 | 5 | 4 |
| 84 | 5 | 5 | 5 | 5 |

Test Example 7C (herbicidal test by soil treatment in an upland farm)

Upland farm soil was filled in plastic pots (120 $cm^2$), and seeds of barnyardgrass, crabgrass, redroot pigweed and rice flatsedge were sown and covered with the soil. Each of the test compounds was formulated into a wettable powder in accordance with Formulation Example 3B. A predetermined amount of the wettable powder was diluted with water, and by a small-sized sprayer, uniformly sprayed onto the soil surface at a rate of 1000 liters/hectare (the amount applied: 4 kg per hectare as the active ingredient). The plants were grown for 20 days in a greenhouse after the treatment, and the herbicidal efficacy and phytotoxicity were examined in accordance with the standards given in Table 1C. The results are shown in Table 8C.

Table 8C

| Test compound No. | Herbicidal efficacy | | | |
|---|---|---|---|---|
| | barn-yard-grass | crab-grass | redroot pigweed | rice flats-edge |
| 4 | 5 | 5 | 4 | 5 |
| 82 | 5 | 5 | 5 | 5 |
| 83 | 5 | 2 | 5 | 5 |
| 84 | 5 | 5 | 5 | 5 |

Test Example 8C (herbicidal efficacy and phytotoxicity by soil treatment in an upland farm

Upland farm soil was filled in plastic vats (600 $cm^2$). Seeds of barnyardgrass, crabgrass, green foxtail, redroot pigweed, rice flatsedge, rice, wheat, corn, soybeans, cotton and sugar beet were sown and covered with the soil. Each of the test compounds was formulated into a wettable powder in accordance with Formulation Example 3B. A predetermined amount of the wettable powder was diluted with water and uniformly sprayed onto the soil surface by a small-sized sprayer at a rate of 1000 liters per hectare. The plants were grown in a greenhouse for 30 days after the treatment. The herbicidal efficacy and phytotoxicity were examined in accordance wiht the standards given in Table 1C. The results are shown in Table 9C.

Table 9C

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flats-edge | rice | wheat | corn | soy beans | cotton | sugar beet |
| 84 | 1 | 5 | 5 | 5 | 5 | 5 | 3 | 5 | 5 | 1.5 | 3.5 | 2 |
| | 0.5 | 5 | 5 | 5 | 5 | 5 | 2 | 3 | 2 | 0.5 | 1.5 | 1 |
| | 0.25 | 5 | 5 | 5 | 4 | 5 | 1 | 0.5 | 0 | 0 | 0.5 | 1 |

Test Example 9C (herbicidal test by soil treatment in a paddy)

Porcelain pots, 10 cm in diameter, were filled with paddy soil, and after puddling, seeds of barnyardgrass, umbrella plant, monochoria and bulrush were sown. The pots were then watered to a depth of 3 cm. On the next day, a wettable powder prepared in accordance with Formulation Example 3B was diluted with water and dropped onto the water surface (amount applied; 4 kg per hectare as the active ingredient). The plants were then grown in a greenhouse, and 30 days after the treatment, the herbicidal activity of the compound of the invention was examined according to the standards given in Table 1C. The results are shown in Table 10C.

Table 10C

| Test compound No. | Herbicidal efficacy | | | |
|---|---|---|---|---|
| | barn-yard-grass | umbrella plant | monochoria | bulrush |
| 85 | 0 | 0 | 5 | 0 |
| 86 | 5 | 5 | 5 | 5 |
| 87 | 5 | 5 | 5 | 5 |
| 88 | 5 | 5 | 5 | 5 |
| 90 | 1 | 5 | 5 | 4 |
| 91 | 5 | 5 | 5 | 5 |
| 93 | 4 | 5 | 4 | 4 |

Test Example 10C (herbicidal test by soil treatment in an upland farm

Upland farm soil was filled in plastic pots (120 $cm^2$), and seeds of barnyardgrass, crabgrass, redroot pigweed and rice flatsedge were sown and covered with the soil. Each of the test compounds was formulated into a wettable powder in accordance with Formulation Example 3B. A predetermined amount of the wettable powder was diluted with water, and by a small-sized sprayer, uniformly sprayed onto the soil surface at a rate of 1000 liters/hectare (the amount applied: 4 kg per hectare as the active ingredient). The plants were grown for 20 days in a greenhouse after the treatment, and the herbicidal efficacy and phytotoxicity were examined in accordance with the standards given in Table 1C. The results are shown in Table 11C.

Table 11C

| Test compound No. | Herbicidal efficacy | | | |
|---|---|---|---|---|
| | barn-yard-grass | crab-grass | redroot pigweed | rice flats-edge |
| 86 | 5 | 5 | 4 | 5 |
| 87 | 5 | 5 | 5 | 5 |
| 88 | 5 | 5 | 5 | 5 |
| 89 | 0 | 1 | 0 | 5 |
| 90 | 3 | 5 | 5 | 5 |
| 91 | 5 | 5 | 5 | 5 |
| 92 | 0 | 0 | 2 | 5 |
| 93 | 5 | 5 | 5 | 5 |

Test Example 11C (herbicidal efficacy and phytotoxicity by soil treatment in an upland farm

Upland farm soil was filled in plastic vats (600 $cm^2$). Seeds of barnyardgrass, crabgrass, green foxtail, redroot pigweed, rice flatsedge, rice, wheat, corn, soybeans, cotton and sugar beet were sown and covered with the soil. Each of the test compounds was formulated into a wettable powder in accordance with Formulation Example 3B. A predetermined amount of the wettable powder was diluted with water and uniformly sprayed onto the soil surface by a small-sized sprayer at a rate of 1000 liters per hectare. The plants were grown in a greenhouse for 30 days after the treatment. The herbicidal efficacy and phytotoxicity were examined in accordance with the standards given in Table 1C. The results are shown in Table 12C.

**Table 12C**

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flats-edge | rice | wheat | corn | soy beans | cotton | sugar beet |
| 86 | 1 | 5 | 5 | 5 | 5 | 5 | 4.5 | 1 | 4.5 | 0.5 | 4 | 2 |
| | 0.5 | 5 | 5 | 5 | 5 | 5 | 4.5 | 0.5 | 4.5 | 0 | 4 | 1.5 |
| | 0.25 | 5 | 5 | 5 | 4.5 | 5 | 4 | 0 | 4.5 | 0 | 4 | 1.5 |
| 87 | 1 | 5 | 5 | 5 | 5 | 5 | 0 | 0.5 | 1.5 | 0 | 1 | 2 |
| | 0.5 | 5 | 5 | 5 | 5 | 5 | 0 | 0 | 1 | 0 | 0 | 1 |
| | 0.25 | 5 | 5 | 5 | 5 | 5 | 0 | 0 | 0 | 0 | 0 | 0 |
| 88 | 1 | 5 | 5 | 5 | 4.5 | 5 | 0.5 | 0.5 | 2 | 1 | 0 | 5 |
| | 0.5 | 5 | 5 | 5 | 3 | 5 | 0 | 0 | 2 | 0.5 | 0 | 2 |
| | 0.25 | 5 | 5 | 5 | 2 | 5 | 0 | 0 | 1.5 | 0.5 | 0 | 0 |
| 93 | 2 | 5 | 5 | 5 | 2 | 5 | 0 | 0 | 0 | 0 | 0 | 0 |
| | 1 | 5 | 5 | 5 | 0 | 5 | 0 | 0 | 0 | 0 | 0 | 0 |
| | 0.5 | 3 | 4 | 4.5 | 0 | 4.5 | 0 | 0 | 0 | 0 | 0 | 0 |
| Comparison* | 1 | 2.5 | 4.5 | 4 | 5 | 5 | 1 | 1 | 2 | 1 | 0 | 5 |
| | 0.5 | 1 | 3.5 | 3 | 5 | 5 | 0.5 | 0.5 | 1.5 | 0 | 0 | 5 |
| | 0.25 | 0 | 1 | 1 | 4 | 1 | 0 | 0 | 0 | 0 | 0 | 4.5 |

*: fluomethuron 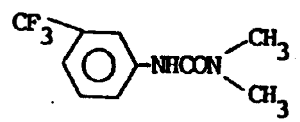

## Claims

1. A substituted aryloxyurea represented by the following formula (I)

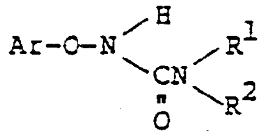 (I)

wherein Ar represents a group selected from the class consisting of aryl groups of the following formulae

(a)

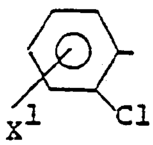

in which $X^1$ represents a hydrogen atom, a $C_1$-$C_4$ alkyl group or a $C_1$-$C_4$ alkoxy group,

(b)

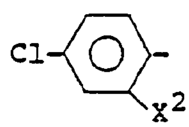

in which $X^2$ represents a hydrogen or halogen atom,

(c)

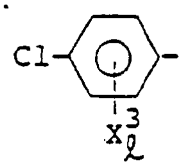

in which $X^3$ represents a halogen atom and $\ell$ is an integer of 2 or 3,

(d)

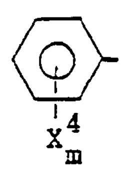

in which $X^4$ represents a fluorine, bromine or iodine atom, and m is an integer of 1, 2 or 3,

(e)

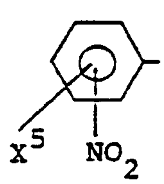

in which $X^5$ represents a hydrogen atom, a halogen atom, a $C_1$-$C_4$ alkyl group, a $C_1$-$C_4$ alkoxy group or a trifluoromethyl group

(f)

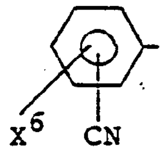

in which $X^6$ represents a hydrogen or halogen atom,

(g)

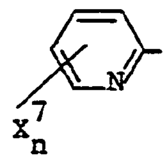

in which $X^7$ represents a hydrogen atom, a halogen atom, a cyano group, a $C_1$-$C_4$ alkoxy group or a trifluoromethyl group, and n is an integer of 1 or 2,

(h)

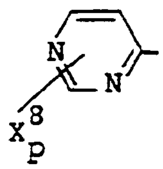

in which $X^8$ represents a hydrogen atom, a chlorine atom or a $C_1$-$C_4$ alkylthio group, and p is an integer of 1 or 2,

(i)

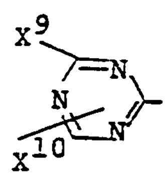

in which $X^9$ and $X^{10}$ are identical or different and each represents a chlorine atom, a $C_1$-$C_4$ alkoxy group or a $C_1$-$C_4$ alkylthio group, and

(j)

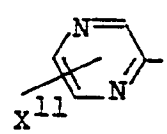

in which $X^{11}$ represents a hydrogen or chlorine atom;
$R^1$ represents a $C_1$-$C_6$ alkyl group, a $C_3$-$C_5$ alkenyl group, a $C_3$-$C_5$ alkynyl group or a $C_3$-$C_7$ cycloalkyl group; $R^2$ represents a hydrogen atom or a $C_1$-$C_6$ alkyl group; and $R^1$ and $R^2$, together with the nitrogen atom to which they are bonded, may form a 4- to 8-membered heterocyclic ring which may contain an oxygen atom as a ring member or may be substituted by a $C_1$-$C_4$ alkyl group or a $C_2$-$C_4$ alkylene group, with the proviso that Ar does

not represent 2,5-difluorophenyl, when $R^1$ is cyclopentyl or cyclohexyl and $R^2$ is and acid hydrogen atom, and acid addition salts thereof.

2. The compounds set forth in claim 1 wherein in formula (I), Ar is selected from the groups of formulae (a), (d), (e), (g), (h) and (j), $R^1$ represents a $C_1$-$C_6$ alkyl or $C_3$-$C_7$ cycloalkyl group, $R^2$ represents a $C_1$-$C_6$ alkyl group, or $R^1$ and $R^2$, taken together, form a 6- to 7-membered heterocyclic group together with the nitrogen atom to which they are bonded.

3. The compounds set forth in claim 1 wherein in formula (I), Ar is the group of formula (g), and $R^1$ and $R^2$, taken together, form a 6- to 7-membered heterocyclic group together with nitrogen atom to which they are bonded.

4. The compounds set forth in claim 1 wherein in formula (I), Ar is a 6-chloro-2-pyridyl or 6-trifluoromethyl-2-pyridyl group, and

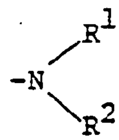 is 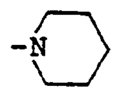, 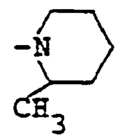,

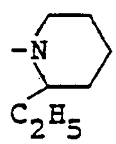, or 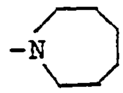.

5. A process for producing a substituted aryloxy-urea represented by the following formula (I)-1

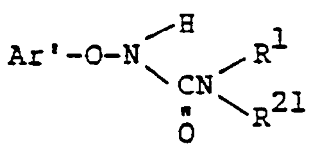 ..... (I)-1

wherein Ar′ is a group selected from the class consisting of the aryl groups of formulae (a) to (g) defined as Ar in formula (I), $R^1$ is as defined with regard to formula (I), $R^{21}$ represents a $C_1$-$C_6$ alkyl group, and $R^1$ and $R^{21}$ may form the same heterocyclic group as the 4- to 8-membered heterocyclic group formed by $R^1$ and $R^2$ in formula (I), which comprises reacting an aryloxyamine represented by the following formula (II)

$$Ar'\text{-}ONH_2 \quad (II)$$

wherein Ar′ is as defined above, with a carbamic acid chloride represented by the following formula (III)

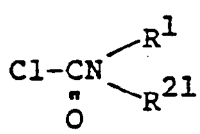 (III)

wherein $R^1$ and $R^{21}$ are as defined above with regard to formula (I)-1, in the presence of a base.

6. A process for producing a substituted aryloxy-urea represented by the following formula (I)-2

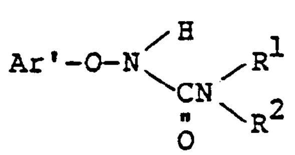 (I)-2

wherein Ar′ is as defined above with regard to formula (II), and $R^1$ and $R^2$ are as defined with regard to formula (I), with the proviso of claim 1, which comprises reacting an aryloxycarbamic acid ester represented by the following formula (IV)

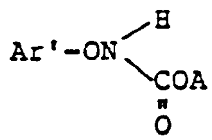

(IV)

wherein Ar' is as defined with regard to formula (II), and A represents a substituted or unsubstituted phenyl group,
with an amine represented by the following formula (V)

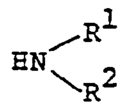

(V)

wherein $R^1$ and $R^2$ are as defined above.

7. A process for producing a substituted aryloxy-urea represented by the following formula (I)-3

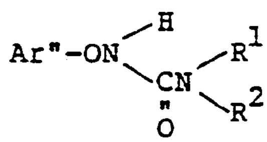

(I)-3

wherein Ar" is a group selected from the class consisting of the aryl groups of formulae (h), (i) and (j) in formula (I), and $R^1$ and $R^2$ are as defined with regard to formula (I),
which comprises reacting a chloride selected from the group consisting of chlorinated pyrimidines represented by the following formula (VI)

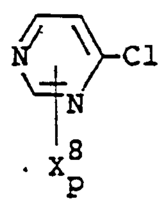

(VI)

wherein $X^8$ and p are as defined with regard to formula (I),
chlorinated triazines represented by the following formula (VII)

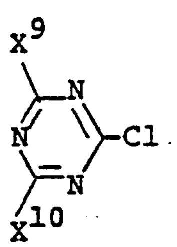

(VII)

wherein $X^9$ and $X^{10}$ are as defined with regard to formula (I),
and chlorides represented by the following formula (VIII)

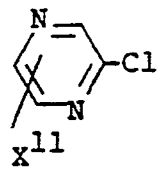

(VIII)

wherein $X^{11}$ is as defined with regard to formula (I), with an N-hydroxyurea represented by the following formula (IX)

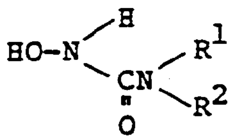

(IX)

wherein $R^1$ and $R^2$ are as defined with regard to formula (I), in the presence of a base.

8. A herbicide comprising a substituted aryloxy-urea represented by formula (I) above or its acid addition salt as an active ingredient.

**Patentansprüche**

1. Substituierter Aryloxyharnstoff, dargestellt durch die folgende Formel (I)

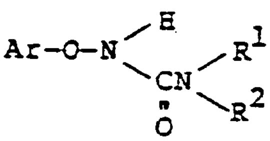

(I)

wobei Ar für eine Gruppe steht, ausgewählt aus der Klasse, bestehend aus Arylgruppen der folgenden Formeln

(a)

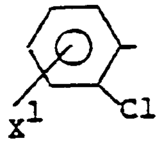

in der $X^1$ für ein Wasserstoffatom, eine $C_{1-4}$-Alkylgruppe oder eine $C_{1-4}$-Alkoxygruppe steht,

(b)

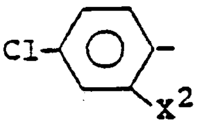

in der $X^2$ für ein Wasserstoff- oder Halogenatom steht,

(c)

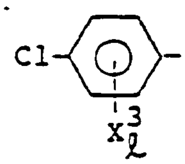

in der $X^3$ für ein Halogenatom steht und eine ganze Zahl von 2 oder 3 ist,

(d)

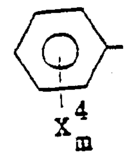

in der $X^4$ für ein Fluor-, Brom- oder Jodatom steht und m eine ganze Zahl von 1, 2 oder 3 ist,

(e)

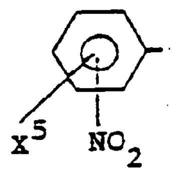

in der $X^5$ für ein Wasserstoffatom, ein Halogenatom, eine $C_{1-4}$-Alkylgruppe, eine $C_{1-4}$-Alkoxygruppe oder eine Trifluormethylgruppe steht,

(f)

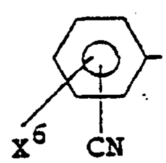

in der $X^6$ für ein Wasserstoff- oder Halogenatom steht.

(g)

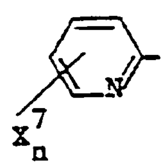

in der $X^7$ für ein Wasserstoffatom, ein Halogenatom, eine Cyanogruppe, eine $C_{1-4}$-Alkoxygruppe oder eine Trifluormethylgruppe steht und n eine ganze Zahl von 1 oder 2 ist,

(h)

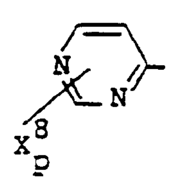

in der $X^8$ für ein Wasserstoffatom, ein Chloratom oder eine $C_{1-4}$-Alkylthiogruppe steht und p eine ganze Zahl von 1 oder 2 ist,

(i)

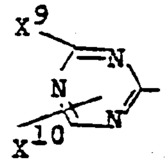

in der $X^9$ und $X^{10}$ identisch oder verschieden sind und jeweils für ein Chloratom, eine $C_{1-4}$-Alkoxygruppe oder eine $C_{1-4}$-Alkylthiogruppe stehen, und

(j)

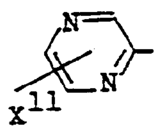

in der $X^{11}$ für ein Wasserstoff- oder Chloratom steht;
R1 für eine $C_{1-6}$-Alkylgruppe, eine $C_{3-5}$-Alkenylgruppe, eine $C_{3-5}$-Alkinylgruppe oder eine $C_{3-7}$-Cycloalkylgruppe steht;
$R^2$ für ein Wasserstoffatom oder eine $C_{1-6}$-Alkylgruppe steht; und $R^1$ und $R^2$, zusammen mit dem Stickstoffatom, an das sie gebunden sind, einen 4- bis 8- gliedrigen, heterocyclischen Ring bilden können, der ein Sauerstoffatom als ein Ringglied enthalten kann oder substituiert sein kann durch eine $C_{1-4}$-Alkylgruppe oder eine $C_{2-4}$-Alkylengruppe, mit der Maßgabe, daß Ar nicht für 2,5-Difluorphenyl steht, falls $R^1$ Cyclopentyl oder Cyclohexyl ist und $R^2$ ein Wasserstoffatom ist, und Säureadditionssalze derselben.

2. Verbindungen gemäß Anspruch 1, wobei in Formel (I) Ar ausgewählt ist aus den Gruppen der Formeln (a), (d), (e), (g), (h) und (j), $R^1$ für eine $C_{1-6}$-Alkyl- oder $C_{3-7}$-Cycloalkylgruppe steht, $R^2$ für eine $C_{1-6}$-Alkylgruppe steht, oder $R^1$ und $R^2$ zusammengefaßt eine 6- bis 7-gliedrige heterocyclische Gruppe bilden, zusammen mit dem Stickstoffatom, an das sie gebunden sind,

3. Verbindungen gemäß Anspruch 1, wobei in Formel (I) Ar für die Gruppe der Formel (g) steht und $R^1$ und $R^2$ zusammengefaßt eine 6- bis 7-gliedrige heterocyclische Gruppe bilden, zusammen mit dem Stickstoffatom, an das sie gebunden sind.

4. Verbindungen gemäß Anspruch 1, wobei in Formel (I) Ar für eine 6-Chlor-2-pyridyl- oder 6-Trifluormethyl-2-pyridyl Gruppe steht und

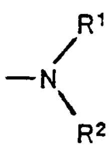 für 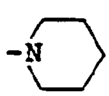, 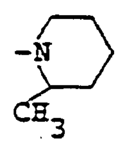, 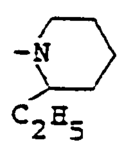 oder 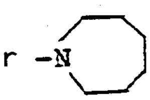 steht.

5. Verfahren zur Herstellung eines substituierten Aryloxyharnstoffs, dargestellt durch die folgende Formel (I)-1

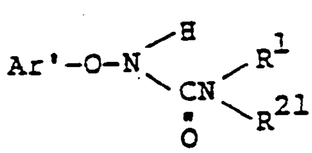 (I)-1

wobei Ar' für eine Gruppe steht, ausgewählt aus der Klasse, bestehend aus den Arylgruppen der Formeln (a) bis (g), wie sie als Ar in Formel (I) definiert sind, $R^1$ wie bei Formel (I) definiert ist, $R^{21}$ für eine $C_{1-6}$ Alkylgruppe steht und $R^1$ und $R^{21}$ die gleiche heterocyclische Gruppe bilden können wie die 4- bis 8-gliedrige heterocy-

clische Gruppe, die durch $R^1$ und $R^2$ in Formel (I) gebildet wird,
umfassend die Umsetzung eines Aryloxyamins der folgenden Formel (II)

$$Ar'\text{-}ONH_2 \quad (II)$$

wobei Ar' wie oben definiert ist, mit einem Carbaminsäurechlorid der folgenden Formel (III)

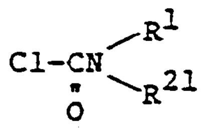

(III)

wobei $R^1$ und $R^{21}$ die oben bei Formel (I)-1 angegebene Bedeutung haben,
in Anwesenheit einer Base.

6. Verfahren zur Herstellung eines substituierten Aryloxyharnstoffs, dargestellt durch die folgende Formel (I)-2

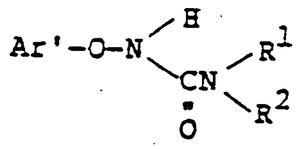

(I)-2

wobei Ar' wie oben bei Formel (II) definiert ist und $R^1$ und $R^2$ wie bei Formel (I) definiert sind,
mit der Maßgabe von Anspruch 1,
umfassend die Umsetzung eines Aryloxycarbaminsäureesters, dargestellt durch die folgende Formel (IV)

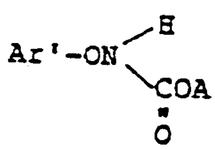

(IV)

wobei Ar' wie bei Formel (II) definiert ist und A für eine substituierte oder unsubstituierte Phenylgruppe steht,
mit einem Amin der folgenden Formel (V)

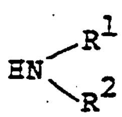

(V)

wobei $R^1$ und $R^2$ wie oben definiert sind.

7. Verfahren zur Herstellung eines substituierten Aryloxyharnstoffs, dargestellt durch die folgende Formel (I)-3

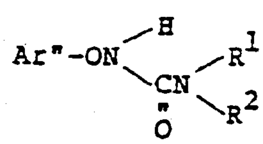

(I)-3

wobei A" für eine Gruppe steht, ausgewählt aus der Klasse, bestehend aus den Arylgruppen der Formeln (h), (i) und (j) in Formel (I), und $R^1$ und $R^2$ wie bei Formel (I) definiert sind,
umfassend die Umsetzung eines Chlorids, ausgewählt aus der Gruppe, bestehend aus chlorierten Pyrimidinen, dargestellt durch die folgende Formel (VI)

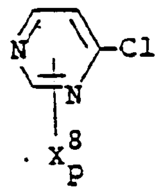

(VI)

wobei $X^8$ und p wie bei Formel (I) definiert sind,
chlorierten Triazinen, dargestellt durch die folgende Formel (VII)

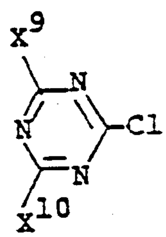

(VII)

wobei $X^9$ und $X^{10}$ wie bei Formel (I) definiert sind,
und Chloriden, dargestellt durch die folgende Formel (VIII)

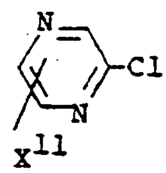

(VIII)

wobei $X^{11}$ wie bei Formel (I) definiert ist,
mit einem N-Hydroxyharnstoff, dargestellt durch die folgende Formel (IX)

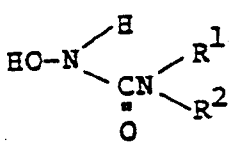

(IX)

wobei $R^1$ und $R^2$ wie bei Formel (I) definiert sind,
in Anwesenheit einer Base.

8. Herbizid, umfassend einen substituierten Aryloxyharnstoff, dargestellt durch die obige Formel (I), oder deren Säureadditionssalz als einen Wirkstoff.

## Revendications

1. Aryloxyurée substituée représentée par la formule (I) :

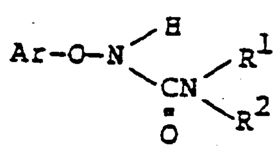

(I)

dans laquelle : Ar représente un groupe choisi dans la classe consistant en les groupes aryle des formules suivantes :

(a)

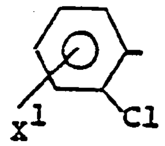

dans laquelle $X^1$ représente un atome d'hydrogène, un groupe alkyle en $C_1$-$C_4$ ou un groupe alcoxy en $C_1$-$C_4$,

(b)

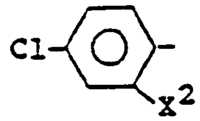

dans laqelle $X^2$ représente un atome d'hydrogène ou d'halogène,

(c)

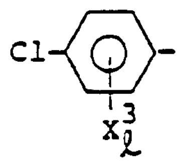

dans laqelle $X^3$ représente un atome d'halogène et $\ell$ est le nombre entier 2 ou 3,

(d)

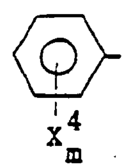

dans laquelle $X^4$ représente un atome de fluor, de brome ou d'iode, et m est le nombre entier de 1, 2 ou 3,

(e)

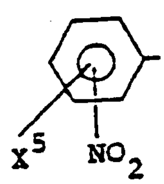

dans laquelle $X^5$ représente un atome d'hydrogène, un atome d'halogène, un groupe alkyle en $C_1$-$C_4$, un groupe alcoxy en $C_1$-$C_4$ ou un groupe trifluorométhyle,

(f)

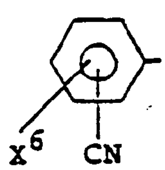

dans laquelle $X^6$ représente un atome d'hydrogène ou d'halogène,

(g)

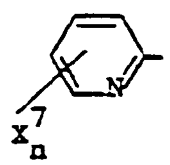

dans laquelle $X^7$ représente un atome d'hydrogène, un atome d'halogène, un groupe cyano, un groupe alcoxy en $C_1$-$C_4$ ou un groupe trifluorométhyle, et n est le nombre entier 1 ou 2,

(h)

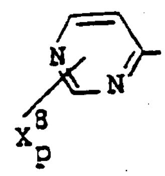

dans laqelle $X^8$ représente un atome d'hydrogène, un atome de chlore ou un groupe alkylthio en $C_1$-$C_4$, et p est le nombre entier 1 ou 2,

(i)

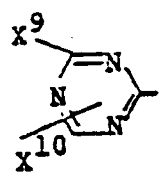

dans laquelle $X^9$ et $X^{10}$ sont identiques ou différents et représentent chacun un atome de chlore, un groupe alcoxy en $C_1$-$C_4$ ou un groupe alkylthio en $C_1$-$C_4$, et

(j)

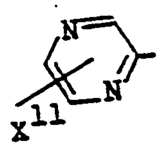

dans laqelle $X^{11}$ représente un atome d'hydrogène ou de chlore ;

- $R^1$ représente un groupe alkyle en $C_1$-$C_6$, un groupe alcényle en $C_3$-$C_5$, un groupe alcynyle en $C_3$-$C_5$ ou un groupe cycloalkyle en $C_3$-$C_7$ ;
- $R^2$ représente un atome d'hydrogène ou un groupe alkyle en $C_1$-$C_6$ ; et - $R^1$ et $R^2$, conjointement avec l'atome d'azote auquel ils sont liés, peuvent former un hétérocycle à 4 à 8 chaînons, qui peut contenir un atome d'oxygène en tant que chaînon du cycle ou peut être substitué par un groupe alkyle en $C_1$-$C_4$ ou un groupe alkylène en $C_2$-$C_4$, avec la condition que Ar ne représente pas difluoro-2,5 phényle, lorsque $R^1$ est cyclopentyle ou cyclohexyle et $R^2$ est un atome d'hydrogène, et les sels d'addition avec un acide de celle-ci.

2. Composés tels que définis à la revendication 1, dans lesquels, dans la formule (I), Ar est choisi parmi les groupes des formules (a), (d), (e), (g), (h) et (j), $R^1$ représente un groupe alkyle en $C_1$-$C_6$ ou cycloalkyle en $C_3$-$C_7$, $R^2$ représente un groupe alkyle en $C_1$-$C_6$, ou $R^1$ et $R^2$, pris ensemble, forment un groupe hétérocyclique à 6 ou 7 chaînons conjointement avec l'atome d'azote auquel ils sont liés.

3. Composés tels que définis à la revendication 1, dans lesquels, dans la formule (I), Ar est le groupe de formule (g), et $R^1$ et $R^2$, pris ensemble, forment un groupe hétérocyclique à 6 ou 7 chaînons conjointement avec l'atome d'azote auquel ils sont liés.

4. Composés tels que définis à la revendication 1, dans lesquels, dans la formule (I), Ar est un group chloro-6 pyridyle-2 ou trifluorométhyl-6 pyridyle-2,

est 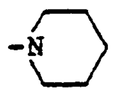, 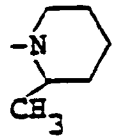, 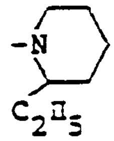, ou 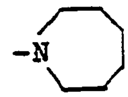. et 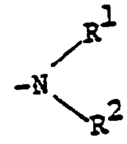

5. Procédé de fabrication d'une aryloxyurée substituée représentée par la formule (I)-1 suivante :

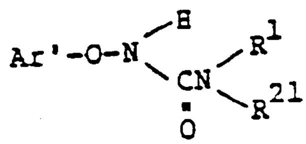 (I)-1

dans laquelle : - Ar' est un groupe choisi dans la classe consistant en les groupes agile des formules (a) à (g) définies en tant que Ar dans la formule (I) ; - $R^1$ est tel que défini en ce qui concerne la formule (I), - $R^{21}$ représente un groupe alkyle en $C_1$-$C_6$, et $R^1$ et $R^{21}$ peuvent former le même groupe hétérocyclique que le groupe hétérocyclique à 4 à 8 chaînons formé par $R^1$ et $R^2$ dans la formule (I), qui comprend la reaction d'une aryloxyamine représentée par la formule (II) suivante :

$$Ar'\text{-}ONH_2 \quad (II)$$

dans laquelle Ar' est tel que défini ci-dessus, avec un chlorure d'acide carbamique représenté par la formule (III) suivante :

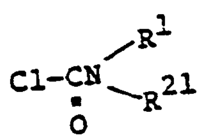 (III)

dans laqelle $R^1$ et $R^{21}$ sont tels que définis ci-dessus en ce qui concerne la formule (I)-1,
en présence d'une base.

6. Procédé de fabrication d'une aryloxyurée substituée représentée par la formule (I)-2 suivante :

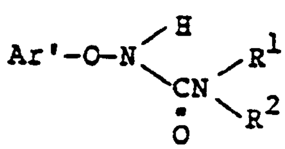 (I)-2

dans laquelle : - Ar' est tel que défini ci-dessus en ce qui concerne la formule (II) ; et - $R^1$ et $R^2$ sont tels que définis en ce qui concerne la formule (I),
avec la condition de la revendication 1,
qui comprend la réaction d'un ester d'acide aryloxycarbamique représenté par la formule (IV) suivante :

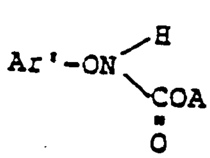 (IV)

dans laquelle : - Ar' est tel que défini en ce qui concerne la formule (II) ; et - A représente un groupe phényle substitué ou non-substitué,
avec une amine représentée par la formule suivante (V) :

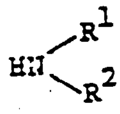

(V)

dans laquelle $R^1$ et $R^2$ sont tels que définis ci-dessus.

7. Procédé de fabrication d'une aryloxyurée substituée représentée par la formule (I)-3 suivante :

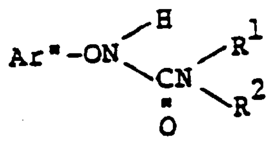

(I)-3

dans laquelle : - Ar″ est un groupe choisi dans la classe consistant en les groupes aryle des formules (h), (i) et (j) dans la formule (I), et - $R^1$ et $R^2$ sont tels que définis en ce qui concerne la formule (I), qui comprend la réaction d'un chlorure choisi dans le groupe constitué par les pyrimidines chlorées représentées par la formule (VI) suivante :

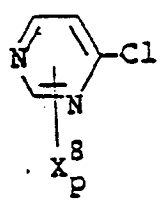

(VI)

dans laquelle $X^8$ et p sont tels que définis en ce qui concerne la formule (I), les triazines chlorées représentées par la formule (VII) suivante :

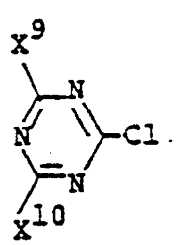

(VII)

dans laquelle $X^9$ et $X^{10}$ sont tels que définis en ce qui concerne la formule (I), et les chlorures représentés par la formule (VIII) suivante:

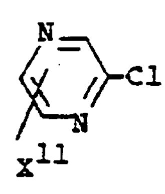

(VIII)

dans laquelle $X^{11}$ est tel que défini en ce qui concerne la formule (I), avec une N-hydroxyurée représentée par la formule (IX) suivante :

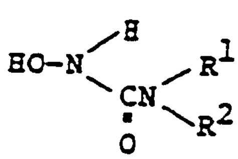

(IX)

dans laquelle $R^1$ et $R^2$ sont tels que définis en ce qui concerne la formule (I), en présence d'une base.

8. Herbicide comprenant une aryloxyurée substitutée représentée par la formule (I) ci-dessus, ou l'un de ses sels d'addition avec un acide, en tant qu'ingrédient actif.